(19) Europäisches Patentamt / European Patent Office / Office européen des brevets

(11) **EP 3 817 764 B1**

(12) **EUROPEAN PATENT SPECIFICATION**

(45) Date of publication and mention of the grant of the patent:
**17.04.2024 Bulletin 2024/16**

(21) Application number: **19768889.8**

(22) Date of filing: **08.07.2019**

(51) International Patent Classification (IPC):
*A61K 38/17* (2006.01)  *A61P 9/10* (2006.01)
*A61P 5/50* (2006.01)  *A61P 3/04* (2006.01)
*A61K 48/00* (2006.01)  *C07K 14/47* (2006.01)
*C07K 14/705* (2006.01)

(52) Cooperative Patent Classification (CPC):
**A61K 38/177; A61P 3/04; A61P 5/50; A61P 9/10; C07K 14/47; C07K 14/705;** C07K 2319/43

(86) International application number:
**PCT/IB2019/055788**

(87) International publication number:
**WO 2020/008442 (09.01.2020 Gazette 2020/02)**

(54) **BIOPHARMACEUTICAL AGENTS FOR USE IN REDUCING LIPID CONTENT IN CELLS**

BIOPHARMAZEUTISCHE MITTEL ZUR SENKUNG DES LIPIDGEHALTS IN ZELLEN

AGENTS BIOPHARMACEUTIQUES DESTINÉS À ÊTRE UTILISÉS DANS LA RÉDUCTION DE LA TENEUR EN LIPIDES DANS DES CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.07.2018 ZA 201804507**
**06.07.2018 ZA 201804509**

(43) Date of publication of application:
**12.05.2021 Bulletin 2021/19**

(73) Proprietor: **University Of The Witwatersrand, Johannesburg**
**Braamfontein**
**Johannesburg 2000 Gauteng (ZA)**

(72) Inventors:
• **WEISS, Stefan Franz Thomas**
**2196 Johannesburg (ZA)**
• **OTGAAR, Tyrone Chad**
**1709 Roodepoort (ZA)**
• **VAN DER MERWE, Eloise**
**1459 Boksburg (ZA)**
• **BERNERT, Martin**
**2191 Johannesburg (ZA)**
• **MORRIS, Gavin**
**2196 Johannesburg (ZA)**

(74) Representative: **Cremer & Cremer**
**Patentanwälte**
**St.-Barbara-Straße 16**
**89077 Ulm (DE)**

(56) References cited:
**WO-A2-2017/077516**    **WO-A2-2017/077516**
**JP-A- 2015 155 383**    **JP-A- 2018 027 935**
**JP-A- 2018 027 935**

Remarks:
The complete document including Reference Table(s) and the Sequence Listing(s) can be downloaded from the EPO website

**Description**

**FIELD OF DISCLOSURE**

This disclosure relates to a method of decreasing lipid concentration in a target cell of a human or animal subject. The disclosure extends to use of biopharmaceutical agents including (i). 37 kDa/67 kDa laminin receptor precursory high affinity laminin receptor (LRP/LR) and/or a fragment thereof, or (ii). a transfecting agent for the expression of LRP/LR, in the treatment and/or prevention of atherosclerosis and/or insulin resistance and/or diabetes in a human or animal subject. In use administration of the biopharmaceutical agent reduces lipid content at target cells.

**BACKGROUND**

Atherosclerosis is a chronic, progressive disease which involves blockage of the arteries, and is the major form of cardiovascular disease (CVD). It is the result of the formation of atherosclerotic plaques which occlude the arteries and decrease blood flow to vital organs.

Atherosclerosis is influenced greatly by oxidative stress, with reactive oxygen species (ROS) contributing largely towards the oxidative state of the body. ROS are formed during various cellular processes, such as cellular respiration. They are produced when an oxygen molecule loses electrons, which allows them to readily partake in oxidative reactions, and thus contribute to oxidative stress (Chen et al., 2003). ROS readily reacts with low-density lipoproteins (LDL) to form oxidised LDL (oxLDL), a molecule responsible for the inflammatory response that results in the formation of atherosclerotic plaques (Grahame and Schlesinger, 2012).

Over time, the accumulation of plaques narrows the blood vessels and occludes the artery, causing the heart to pump harder leading to damaged tissue and hypertension (Tedgui and Mallat, 1999). In addition, these plaques are prone to rupturing due to their soft extracellular lipid components. When these plaques rupture, they can cause complete occlusion of the blood vessels resulting in major cardiovascular events, such as myocardial infarctions or cerebrovascular disease (Tedgui and Mallat, 1999).

Atherosclerosis is the major cause of cardiovascular related deaths, with current therapies such as ACE inhibitors and β-blockers aiming to reduce stress on the cardiac muscle by reducing hypertension caused by the occlusion of the blood vessels (Conte et al., 2015). However, current therapies do not aim to remove the atherosclerotic plaques which are causing the occlusion, and the only current treatments for atherosclerosis is a coronary bypass or expanding of the arteries, both of which are highly invasive. Treatment of atherosclerosis with drugs that lower blood pressure may prevent organs from receiving adequate oxygen and nutrients as the plaques would prevent adequate blood flow, which may cause eventual organ failure (Conte et al., 2015). Furthermore, in the event that these plaques burst, complete occlusion of one or more blood vessels may occur, and depending in the location, could result in major cardiovascular events, such as myocardial infarctions and cerebrovascular events.

Consequently, there is a need for new innovation that will reduce and/or prevent plaque formation.

Atherosclerosis is often co-morbid with obesity, insulin resistance and/or diabetes. A lipid rich diet often plays a major contributory role. Typically, an increase in ROS levels is seen in obesity due to mitochondrial impairment. The molecular mechanism leading to this insulin resistance is triggered by adipose tissue which is metabolically active in obese individuals, thereby continually undergoing lipolysis and generating free fatty acids (FFA) (Gavin et al, 2017). These FFA act as antagonists of insulin (Salpea, 2010), and stimulate the production of glucose by the liver, while suppressing the uptake of glucose by the skeletal muscles. Increased FFA levels decrease the mitochondrial β-oxidation in skeletal muscle cells and liver cells. The reduced mitochondrial β-oxidation results in accumulation of diacylglycerol (DAG) and long-chain acylcoenzyme A (LCCoA) (Savage et al. 2007; Salpea, 2012). This in turn induces serine/threonine phosphorylation of IRS-1 sites, thereby inhibiting IRS-1 phosphorylation and activation of phosphatidylinositol 3-phosphate (PI3P) signaling. PI3P regulates glucose transporter 4 (GLUT4) synthesis (Savage et al. 2007; Salpea, 2012). Thus the inhibition of the activation of PI3P in skeletal results in decreased GLUT4 synthesis and consequently reduced glucose uptake by skeletal muscles. The inhibition of the activation of PI3P in liver cells leads to reduced forkhead box protein O (FOXO) phosphorylation, which in turn results in increased hepatic gluconeogenesis (Savage et al. 2007). Increased FFA in obese individuals leads to hyperglycemia and induction of insulin resistance seen in diabetes type II patients (Gavin, 2017; Mejia, 2006). These cellular changes promote the accumulation of specific lipid metabolites (diacylglycerols and/or ceramides) in liver and skeletal muscle, and lipid content of the cells in diabetes has been found to be increased which is also associated with insulin resistance (Gavin et al, 2017)

Current type II diabetes treatment protocols target blood sugar rather than impaired insulin signaling caused by chronically elevated insulin levels (Ford, 2010). Consequently, there is a need for new innovation that will target impaired insulin signaling.

In WO 2017/077516 A2 (University of the Witwatersrand) compounds for use in the treatment of telomere related diseases, e.g. cancer, are disclosed. Particularly, a 37 kDa/67 kDa laminin receptor precursor / high affinity laminin receptor (LRP/LR) and/or a fragment thereof for use in the treatment and/or prevention of cellular ageing are described. However, not any enabling disclosure for use of such substances in the treatment of obesity, atherosclerosis, insulin resistance and/or diabetes is encompassed.

JP 2018 027935 A describes the effects of oolong tea polymerized polyphenol as an 67LR agonist. It is generally mentioned that 67LR agonists may be used for preventing or treating 67LR-related diseases which may be among others obesity or arteriosclerosis. The examples disclosed mainly refer to cancer. In essence a downregulation of 67LR seems to be the mode of action in JP 2018 027935 A.

JP 2015 155383 A teaches green tea extract having anticancer effects. The tea extract and EGCg are described to enhance the expression of three receptors, the laminin receptor, the retinoid X receptor RXRA and the free fatty acid receptor FFAR2. Activation of the last two receptors are said to "likely have anti-obesity effects".

This disclosure seeks to ameliorate the disadvantages known in the art. The references to methods of treatment in the subsequent paragraphs of this description are to be interpreted as references to the compounds, pharmaceutical compositions and medicaments of the present invention for use in a method for treatment of the human (or animal) body by therapy.

## SUMMARY

The invention is defined in independent claims 1, 10 and 11. The dependent claims relate to preferred embodiments of the invention.

In accordance with a **first,** however not claimed **aspect** of this disclosure there is provided a method of decreasing lipid concentration in a target cell of a human or animal subject, the method comprising the following steps:

(i) transfecting the cell to produce 37 kDa/67 kDa laminin receptor precursor/ high affinity laminin receptor (LRP/LR) and/or a fragment thereof; or
(ii) providing the cell with LRP/LR and/or fragments thereof,

wherein a decrease in lipid concentration in the cell treats and/or prevents atherosclerosis and/or obesity and/or insulin resistance and/or diabetes.

The step of transfecting may include encapsulating a transfecting agent for site specific delivery to the cell, and the step of providing may include encapsulating the LRP/LR for site specific delivery to the cell.

The transfecting agent and/or the LRP/LR and/or the fragment of LRP/LR are biopharmaceutical agents.

Encapsulating may be by means of nanoparticles to form a delivery means for the transfecting agent and/or the LRP/LR. The nanoparticles may be functionalized with chemical, biochemical or biological moieties to ensure site specific delivery to the cell.

The moieties may act as ligands to ensure site specific delivery at the cell.

The cell may be at least one of, but not limited to, the following group: endothelial cells of blood vessels, smooth muscles cells of blood vessels, pancreatic cells including alpha (α) cells, beta (β) cells, delta (δ), and gamma (γ) cells.

The delivery means may be formulated into a pharmaceutical composition, which pharmaceutical composition may further include a pharmaceutical carrier for parenteral or non-parenteral administration to the subject.

Non-parenteral administration may include at least one of, but not limited to, the following group: oral, nasal, rectal, vaginal, optical and transdermal administration. Typically, non-parenteral administration may be oral. Parenteral administration may include at least one of intravenous, subcutaneous and intramuscular administration. Typically, parenteral administration may be intravenous.

The pharmaceutical composition may further include an anti-oxidant such that in use at the target cell the anti-oxidant scavenges reactive oxygen species.

The pharmaceutical composition may further include an active pharmaceutical ingredient (API).

The subject may be a human, animal, reptile, avian, or amphibian. Typically, the subject may be a human and/or animal, preferably human.

The transfecting agent may be pCIneo-moLRP::FLAG plasmid.

LRP/LR may comprise a peptide/protein sequence listing as set forth in SEQ ID NO: 1 or SEQ ID NO: 2, or a fragment thereof.

SEQ ID NO: 1 may be a peptide/protein sequence for human LRP/LR and may have the following sequence:

MSGALDVLQMKEEDVLKFLAAGTHLGGTNLDFQMEQYIYKRKSDGIYIINLKRTWEKLLL

AARAIVAIENPADVSVISSRNTGQRAVLKFAAATGATPIAGRFTPGTFTNQIQAAFREPR

LLVVTDPRADHQPLTEASYVNLPTIALCNTDSPLRYVDIAIPCNNKGAHSVGLMWWMLAR

EVLRMRGTISREHPWEVMPDLYFYRDPEEIEKEEQAAAEKAVTKEEFQGEWTAPAPEFTA

TQPEVADWSEGVQVPSVPIQQFPTEDWSAQPATEDWSAAPTAQATEWVGATTDWS

SEQ ID NO: 2 may be a peptide/protein sequence for mouse (*Mus musculus*) LRP/LR and may have the following sequence:

MSGALDVLQMKEEDVLKFLAAGTHLGGTNLDFQMEQYIYKRKSDGIYIINLKRTWEKLLL

AARAIVAIENPADVSVISSRNTGQRAVLKFAAATGATPIAGRFTPGTFTNQIQAAFREPR

LLVVTDPRADHQPLTEASYVNLPTIALCNTDSPLRYVDIAIPCNNKGAHSVGLMWWMLAR

EVLRMRGTISREHPWEVMPDLYFYRDPEEIEKEEQAAAEKAVTKEEFQGEWTAPAPEFTA

AQPEVADWSEGVQVPSVPIQQFPTEDWSAQPATEDWSAAPTAQATEWVGATTEWS

It is to be understood that LRP/LR is highly conserved and homologs or fragments of SEQ ID NO: 1 and SEQ ID NO: 2, and/or homologs of the fragments may also utilized in order to exercise the invention described, illustrated and/or exemplified herein.

LRP/LR may comprise a peptide/protein sequence listing having at least 80% homology to the sequences as set forth in SEQ ID NO: 1 or SEQ ID NO: 2, or a fragment thereof.

LRP/LR may comprise homologs or fragments thereof, and homologs of the fragments, wherein LRP/LR may comprise a peptide/protein sequence listing as set forth in SEQ ID NO: 1 or SEQ ID NO: 2.

The peptide/protein sequence of LRP/LR or a homolog or fragment thereof, or a homolog of the fragment, may be bound to, or bonded with, or joined to, or conjugated with, or associated with, an additional protein sequence, amino acid sequence, peptide, protein, or antibody. Alternatively and/or additionally, the peptide/protein sequence of LRP/LR may form part of a larger and/or longer peptide/protein sequence. In a certain embodiment of the invention LRP/LR may be may be bound to, or bonded with, or joined to, or conjugated with, or associated with, FLAG protein, such that in use, the LRP/LR may be tagged with FLAG. FLAG protein may include a peptide/protein sequence that includes at least a sequence motif DYKDDDDK (SEQ ID NO:3). Other marker proteins are envisaged aside from FLAG for all embodiments of this disclosure.

It is to be understood that the step of transfecting the cell to produce 37 kDa/67 kDa laminin receptor precursor/ high affinity laminin receptor (LRP/LR) and/or a fragment may take place via known procedures in the art, including introduction into the cell of the transfecting agent. The step of transfecting the cell may upregulate LRP/LR to cause overexpression of LRP/LR.

The fragment of the peptide/protein sequence listing is set forth in SEQ ID NO: 4 corresponding to a fragment of SEQ ID NO: 1 from 102 to 295 and/or SEQ ID NO: 5 corresponding to a fragment of SEQ ID NO: 2 from 102 to 295.

SEQ ID NO: 4 may be a peptide/protein sequence for a fragment of human LRP/LR and may have the following sequence:

RFTPGTFTNQIQAAFREPR

LLVVTDPRADHQPLTEASYVNLPTIALCNTDSPLRYVDIAIPCNNKGAHSVGLMWWMLAR

EVLRMRGTISREHPWEVMPDLYFYRDPEEIEKEEQAAAEKAVTKEEFQGEWTAPAPEFTA

TQPEVADWSEGVQVPSVPIQQFPTEDWSAQPATEDWSAAPTAQATEWVGATTDWS

SEQ ID NO: 5 may be a peptide/protein sequence for a fragment of mouse LRP/LR and may have the following

sequence:

RFTPGTFTNQIQAAFREPR

LLVVTDPRADHQPLTEASYVNLPTIALCNTDSPLRYVDIAIPCNNKGAHSVGLMWWMLAR

EVLRMRGTISREHPWEVMPDLYFYRDPEEIEKEEQAAAEKAVTKEEFQGEWTAPAPEFTA

AQPEVADWSEGVQVPSVPIQQFPTEDWSAQPATEDWSAAPTAQATEWVGATTEWS.

The method extends generally to upregulation of LRP/LR expression in the target cells.

In accordance with a second aspect of this disclosure there is provided a biopharmaceutical agent including a 37 kDa/67 kDa laminin receptor precursor/ high affinity laminin receptor (LRP/LR) and/or a fragment thereof for use in the treatment and/or prevention of atherosclerosis and/or obesity and/or insulin resistance and/or diabetes, wherein LRP/LR and/or the fragment thereof being for administration to a target cell of a subject in need thereof. In use the administration of the biopharmaceutical agent reduces lipid content in the target cell.

LRP/LR may be encapsulated into a delivery means.

The delivery means may include nanoparticles. The nanoparticles may be functionalized with chemical, biochemical or biological moieties to ensure site specific delivery to the target cell.

The moieties may act as ligands to ensure site specific delivery at the target cell.

The target cell may be at least one of, but not limited to, the following group: endothelial cells of blood vessels, smooth muscles cells of blood vessels, pancreatic cells including alpha (α) cells, beta (β) cells, delta (δ), and gamma (γ) cells.

The delivery means may be formulated into a pharmaceutical composition, which pharmaceutical composition may further include a pharmaceutical carrier for parenteral or non-parenteral administration to the subject.

Non-parenteral administration may include at least one of, but not limited to, the following group: oral, nasal, rectal, vaginal, optical and transdermal administration. Typically, non-parenteral administration may be oral. Parenteral administration may include at least one of intravenous, subcutaneous and intramuscular administration. Typically, parenteral administration may be intravenous.

The pharmaceutical composition may further include an anti-oxidant such that in use at the target cell the anti-oxidant scavenges reactive oxygen species.

The pharmaceutical composition may further include an active pharmaceutical ingredient (API).

The subject may be a human, animal, reptile, avian, or amphibian. Typically, the subject may be a human and/or animal, preferably human.

LRP/LR may comprise a peptide/protein sequence listing as set forth in SEQ ID NO: 1 or SEQ ID NO: 2, or comprises a fragment thereof as set out in the first and second aspects herein above.

In accordance with a **third aspect** of this disclosure there is provided a biopharmaceutical agent including a transfecting agent for expressing a 37 kDa/67 kDa laminin receptor precursor/ high affinity laminin receptor (LRP/LR) and/or a fragment thereof, the transfecting agent for use in the treatment and/or prevention of atherosclerosis and/or obesity and/or insulin resistance and/or diabetes, wherein the transfecting agent being for administration to a target cell of a subject in need thereof.

The transfecting agent may be encapsulated into a delivery means.

The delivery means may include nanoparticles. The nanoparticles may be functionalized with chemical, biochemical or biological moieties to ensure site specific delivery to the target cell.

The moieties may act as ligands to ensure site specific delivery at the target cell.

The target cell may be at least one of, but not limited to, the following group: endothelial cells of blood vessels, smooth muscles cells of blood vessels, pancreatic cells including alpha (α) cells, beta (β) cells, delta (δ), and gamma (γ) cells.

The delivery means may be formulated into a pharmaceutical composition, which pharmaceutical composition may further include a pharmaceutical carrier for parenteral or non-parenteral administration to the subject.

Non-parenteral administration may include at least one of, but not limited to, the following group: oral, nasal, rectal, vaginal, optical and transdermal administration. Typically, non-parenteral administration may be oral. Parenteral administration may include at least one of intravenous, subcutaneous and intramuscular administration. Typically, parenteral administration may be intravenous.

The pharmaceutical composition may further include an anti-oxidant such that in use at the target cell the anti-oxidant scavenges reactive oxygen species.

The pharmaceutical composition may further include an active pharmaceutical ingredient (API).

The transfecting agent may be pCIneo-moLRP::FLAG plasmid.

LRP/LR may comprise a peptide/protein sequence listing as set forth in SEQ ID NO: 1 or SEQ ID NO: 2, or comprises a fragment thereof as set out in the first and second aspects herein above.

In accordance with a **fourth aspect** of this disclosure there is provided a pharmaceutical composition comprising a transfecting agent for 37 kDa/67 kDa laminin receptor precursory high affinity laminin receptor (LRP/LR) and/or a fragment thereof and a carrier, the pharmaceutical composition for use in the treatment and/or prevention of atherosclerosis and/or obesity and/or insulin resistance and/or diabetes, wherein LRP/LR and/or the fragment thereof being for administration to a target cell of a subject in need thereof.

The pharmaceutical composition wherein LRP/LR is encapsulated providing a delivery means.

The pharmaceutical composition may further include an anti-oxidant such that in use at the target cell the anti-oxidant scavenges reactive oxygen species.

The pharmaceutical composition may further include an active pharmaceutical ingredient (API).

LRP/LR may comprise a peptide/protein sequence listing as set forth in SEQ ID NO: 1 or SEQ ID NO: 2, or comprises a fragment thereof as set out in the first and second aspects herein above.

In accordance with a **fifth aspect** of this disclosure there is provided a pharmaceutical composition comprising a transfecting agent for expressing 37 kDa/67 kDa laminin receptor precursor/ high affinity laminin receptor (LRP/LR) and/or a fragment thereof, and a carrier, the pharmaceutical composition for use in the treatment and/or prevention of atherosclerosis and/or obesity and/or insulin resistance and/or diabetes.

The pharmaceutical composition wherein the transfecting agent is encapsulated providing a delivery means.

The pharmaceutical composition may further include an anti-oxidant such that in use at the target cell the anti-oxidant scavenges reactive oxygen species.

The pharmaceutical composition may further include an active pharmaceutical ingredient (API).

The subject may be a human, animal, reptile, avian, or amphibian. Typically, the subject may be a human and/or animal, preferably human.

LRP/LR may comprise a peptide/protein sequence listing as set forth in SEQ ID NO: 1 or SEQ ID NO: 2, or comprises a fragment thereof as set out in the first and second aspects herein above.

The transfecting agent may be pCIneo-moLRP::FLAG plasmid.

In accordance with a **sixth,** however not claimed **aspect** of this disclosure there is provided a method of treating and/or preventing atherosclerosis and/or obesity and/or insulin resistance and/or diabetes, the method including the following steps:

(i). transfecting the cell to produce 37 kDa/67 kDa laminin receptor precursory high affinity laminin receptor (LRP/LR) and/or a fragment thereof; or

(ii). providing the cell with LRP/LR and/or fragments thereof,

such that in use there is provided a decrease in lipid concentration in a target cell therein treating and/or preventing atherosclerosis and/or insulin resistance and/or diabetes.

The transfecting agent and/or the LRP/LR and/or the fragment of LRP/LR are biopharmaceutical agents.

The step of transfecting may include encapsulating a transfecting agent for site specific delivery to the cell, and the step of providing may include encapsulating the LRP/LR for site specific delivery to the cell.

Encapsulating may be by means of nanoparticles to form a delivery means for the transfecting agent and/or the LRP/LR. The nanoparticles may be functionalized with chemical, biochemical or biological moieties to ensure site specific delivery to the cell.

The moieties may act as ligands to ensure site specific delivery at the cell.

The targets cell may be at least one of, but not limited to, the following group: endothelial cells of blood vessels, smooth muscles cells of blood vessels, pancreatic cells including alpha ($\alpha$) cells, beta ($\beta$) cells, delta ($\delta$), and gamma ($\gamma$) cells.

The delivery means may be formulated into a pharmaceutical composition, which pharmaceutical composition may further include a pharmaceutical carrier for parenteral or non-parenteral administration to the subject.

Non-parenteral administration may include at least one of, but not limited to, the following group: oral, nasal, rectal, vaginal, optical and transdermal administration. Typically, non-parenteral administration may be oral. Parenteral administration may include at least one of intravenous, subcutaneous and intramuscular administration. Typically, parenteral administration may be intravenous.

The pharmaceutical composition may further include an anti-oxidant such that in use at the target cell the anti-oxidant scavenges reactive oxygen species.

The pharmaceutical composition may further include an active pharmaceutical ingredient (API).

The subject may be a human, animal, reptile, avian, or amphibian. Typically, the subject may be a human and/or animal, preferably human.

The transfecting agent may be pCIneo-moLRP::FLAG plasmid.

LRP/LR may comprise a peptide/protein sequence listing as set forth in SEQ ID NO: 1 or SEQ ID NO: 2, or a fragment thereof as set out in the first aspect of this disclosure herein above.

In accordance with a **seventh aspect** of this disclosure there is provided use of (i). 37 kDa/67 kDa laminin receptor precursor/ high affinity laminin receptor (LRP/LR) and/or a fragment thereof, or (ii). a transfecting agent for the expression of LRP/LR, in the manufacture of a pharmaceutical composition for the treatment and/or prevention of atherosclerosis and/or obesity and/or insulin resistance and/or diabetes.

In accordance with an **eighth aspect** of this disclosure there is provided a pharmaceutical composition for the site specific delivery of a biopharmaceutical agent to a specific target site within a human or animal, the pharmaceutical composition including:

the biopharmaceutical agent encapsulated by a carrier matrix, such that in use the carrier matrix facilitates site specific delivery of the biopharmaceutical and concomitantly hindering degradation thereof prior to reaching, or at, the target site,

wherein the biopharmaceutical agent is at least one of the following group: (i). 37 kDa/67 kDa laminin receptor precursor/ high affinity laminin receptor (LRP/LR) and/or a fragment thereof, (ii). a transfecting agent for the expression of LRP/LR, and (iii). an anti-LRP/LR specific antibody.

The biopharmaceutical agent may additionally or alternatively be any means for upregulating or downregulating LRP/LR expression within the human or animal body. The biopharmaceutical agent may be nucleic acid. The biopharmaceutical agent may be, for example, be siRNA and/or shRNA.

The carrier matrix may be a polymeric carrier matrix.

The polymeric carrier matrix may include polymeric nanoparticles.

The polymeric nanoparticles may include synthetic or natural polymers.

The polymeric nanoparticles may be biodegradable to provide in use a reduced risk of an immunogenic response to said polymeric nanoparticles.

The polymeric nanoparticles may be biocompatible to mitigate risk of any immunogenic response to said polymeric nanoparticles.

The polymeric nanoparticles may be stimuli responsive such that in use the nanoparticles undergo a conformational change upon exposure to certain stimuli to facilitate providing the biopharmaceutical agent to its target site. The nanoparticles may be responsive to stimuli including for example: pH, temperature, and electric current.

The polymeric nanoparticles may be hydrophobic or hydrophilic depending on the specific target site.

The polymeric nanoparticles may be crosslinked. Typically, a crosslinking agent is used for crosslinking. However, crosslinking may take place by way of ultra-violet (U.V.) irradiation.

The polymeric nanoparticles may be lyophilized. Lyophilization typically provides porosity to facilitate diffusion of the biopharmaceutical away from the nanoparticle capsule and to the target site.

The polymeric nanoparticles may be any one or more selected from, but not limited to, the following group: eudragit, gum arabic, carrageenan, cellulose, hydroxypropyl cellulose (HPC), methylcellulose (MC), hydroxypropylmethylcellulose (HPMC), polylactic-co-glycolic acid (PLGA), chitin, pectin, amylopectic, natural rubber, polyethylene, polypropylene, polystyrene, polyamide, polyacrylonitrile, polyvinyl chloride, polyvinyl alcohol (PVA), polyethylene glycol (PEG), polyethylene oxide (PEG), poly(D-lactide) (PDLA), polylactic acid (PLLA), polygalacturonate, methylcellulose (polyacetals), poly($\varepsilon$-caprolactone), phospholipids, polysaccharides, polyanionic polysaccharides, carboxymethyl cellulose, carboxymethyl amylose, chondroitin-6-sulfate, dermatin sulfate, heparin, heparin sulfate, poly(hydroxyethyl methylacrylate), collagen, fibrinogen, albumin, fibrin, acrylamide, hydroxypropyl methacrylamide-based copolymers, polyacrylamide, poly(N-isopropyl acrylamide) (pNIPAAm), polyvinylpyrrolidone, poly(methacrylic acid-g-ethylene glycol), poly(N-2-hydroxypropyl methacrylamide), poly(glycolic acid) (PGA), poly(lactic acid) (PLA), chitosan, poly(2-hydroxyethylmethacrylate) (HEMA), polyphazene, phosphorylcholine, hyaluronic acid (HA), hydroxyethyl methacrylate (HEMA), methylene-bis-acrylamide (MBAAm), poly(acrylic acid) (PAAc), poly-acrylamide (PAAm), polyacrylonitrile (PAN), polybutylene oxide (PBO), polycaprolactone (PCL), polyethylene imine) (PEI), poly(ethyl methacrylate) (PEMA), propylene fumarate (PF), poly(glucosylethyl methacrylate) (PGEMA), poly(hydroxy butyrate) (PHB), poly(hydroxyethyl methacrylate) (PHEMA), poly(hydroxypropyl methacrylamide) (PHPMA), poly(methyl methacrylate) (PMMA), poly(N-vinyl pyrrolidone) (PNVP), polypropylene oxide) (PPO), poly(vinyl acetate) (PVAc), poly(vinyl amine), chondroitin sulfate, dextran sulfate, polylysine, gelatin, carboxymethyl chitin, dextran, agarose, pullulan, polyesters, PEG-PLA-PEG, PEG-PLGA-PEG, PEG-PCL-PEG, PLA-PEG-PLA, poly(PF-co-EG), poly(PEG/PBO-terephthalate), PEG-bis-(PLA-acrylate), PEG6CDs, PEG-g-poly(AAm-co-vinlyamine), poly(NIPAAm-co-AAc), poly(NIPAAm-co-EMA), PNVP, poly(MMA-co-HEMA), poly(AN-co-allyl sulfonate), poly(biscarboxy-phenoxy-phosphazene), poly(GEMA-sulfate), poly(PEG-co-peptides), alginate-g-(PEO-PPO-PEO), poly(PLGA-co-serine), collagen-acrylate, alginate, alginate-acrylate, poly(HPMA-g-peptide), HA-g-NIPAAm, and poly(vinyl methyl ether) (PVME), and/or derivatives of any one or more of the aforementioned.

In an example embodiment of the disclosure the polymeric nanoparticles may be poly(lactic-co-glycolic acid) (PLGA) nanoparticles.

PLGA is biodegradable, biocompatible and can cross the blood brain barrier (BBB).

The nanoparticles may be functionalized with a first functional group. The first functional group may be at least one of, but not limited to, the following group: chemical, biochemical and biological moieties.

Chemical moieties may include organic, inorganic or a combination of organic and inorganic moieties.

Biochemical moieties may include at least one of, but not limited to, the following group: amino acids, peptides, polypeptides, oligopeptides, proteins, enzymes, anti-bodies and RNA or DNA sequences coding for any one of the aforementioned.

Typically, the first functional group in use acts as a ligand to facilitate site specific delivery of the pharmaceutical composition. It is to be understood that the functional groups may differ depending on the target site. In use, the first functional group bonds with, or joins to, or conjugates with, or associates with, the target site.

Additionally, or alternatively, there is provided for the biopharmaceutical to include at least one second functional group.

The second functional group may be at least one of, but not limited to, the following group: chemical, biochemical and biological moieties. In this manner, not only will the nanoparticles aid site specific delivery, but the biopharmaceutical will also aid site specific delivery.

Chemical moieties may include organic, inorganic or a combination of organic and inorganic moieties.

Biochemical moieties may include at least one of, but not limited to, the following group: amino acids, peptides, polypeptides, oligopeptides, proteins, enzymes, antibodies and RNA or DNA sequences coding for any one of the aforementioned. In use, the second functional group bonds with, or joins to, or conjugates with, or associates with, the target site.

The specific target site may be a cell including at least one of, but not limited to, the following group: brain cells, endothelial cells of blood vessels, smooth muscles cells of blood vessels, pancreatic cells including alpha ($\alpha$) cells, beta ($\beta$) cells, delta ($\delta$), and gamma ($\gamma$) cells.

The pharmaceutical composition may further include a pharmaceutical carrier for parenteral or non-parenteral administration to the subject.

Non-parenteral administration may include at least one of, but not limited to, the following group: oral, nasal, rectal, vaginal, optical and transdermal administration. Typically, non-parenteral administration may be oral.

Parenteral administration may include at least one of intravenous, subcutaneous and intramuscular administration. Typically, parenteral administration may be intravenous.

The pharmaceutical composition may further include an anti-oxidant such that in use at the target site the anti-oxidant scavenges reactive oxygen species.

The pharmaceutical composition may further include an active pharmaceutical ingredient (API). The active pharmaceutical ingredient (API) may be included to treat and/or prevent comorbid conditions such as but not limited to: inflammation and pain.

The API may be at least one of, but not limited to, the following group: enfuvirtide; octreotide; cyclosporine; insulin; glucagon; glucagon-like peptide-1 (GLP-1); antibiotics including ciprofloxacin or other fluoroquinolones; peptide antibiotics such as polymixin and colistin; bovine serum albumin (BSA); felodipine, nimodipine; interferon beta; salmon calcitonin; eel calcitonin; chicken calcitonin; rat calcitonin; human calcitonin; porcine calcitonin or any gene-variant of calcitonin; parathyroid hormone; parathyroid hormone analogue PTH 1-31NH$_2$; parathyroid hormone analogue PTH 1-34NH$_2$; insulin of any gene variant; vasopressin; desmopressin; buserelin; luteinizing hormone-releasing factor; erythropoietin; tissue plasminogen activators; human growth factor; adrenocorticototropin; various interleukins; encephalin; etanercept; adalimumab; rituximab; infliximab; abatacept; traztuzumab; feglymycin; heparin; as well as all known vaccines.

The API may also be an analgesic such as acetaminophen or a non-steroidal anti-inflammatory drug (NSAID). NSAIDs include aspirin (Anacin, Ascriptin, Bayer, Bufferin, Ecotrin, Excedrin), choline and magnesium salicylates (CMT, Tricosal, Trilisate), choline salicylate (Arthropan), celecoxib (Celebrex), diclofenac potassium (Cataflam), diclofenac sodium (Voltaren, Voltaren XR), diclofenac sodium with misoprostol (Arthrotec), diflunisal (Dolobid), etodolac (Lodine, Lodine XL), fenoprofen calcium (Nalfon), flurbiprofen (Ansaid), ibuprofen (Advil, Motrin, Motrin IB, Nuprin), indomethacin (Indocin, Indocin SR), ketoprofen (Actron, Orudis, Orudis KT, Oruvail), magnesium salicylate (Arthritab, Bayer Select, Doan's Pills, Magan, Mobidin, Mobogesic), meclofenamate sodium (Meclomen), mefenamic acid (Ponstel), meloxicam (Mobic), nabumetone (Relafen), naproxen (Naprosyn, Naprelan), naproxen sodium (Aleve, Anaprox), oxaprozin (Daypro), piroxicam (Feldene), rofecoxib (Vioxx), salsalate (Amigesic, Anaflex 750, Disalcid, Marthritic, Mono-Gesic, Salflex, Salsitab), sodium salicylate (various generics), sulindac (Clinoril), tolmetin sodium (Tolectin) and Valdecoxib (Bextra).

The pharmaceutical composition may further comprise an inhibitor of cytochrome P450 3A4 (CYP3A4). The inhibitor of cytochrome P450 3A4 (CYP3A4) may be selected from the group consisting of polyethylene glycol, polyamine, polymethyl methacrylate and derivatives thereof, wherein the inhibitor is present in an amount which is effective to substantially inhibit the biopharmaceutical and/or the API from being pre-systemically metabolized resulting in greater bioavailability of the biopharmaceutical and/or API.

The pharmaceutical composition may further comprise a P-glycoprotein (P-gp) efflux pump inhibitor.

In a certain embodiment, wherein the pharmaceutical composition is formulated for oral delivery, the pharmaceutical composition may further include a coating there around, preferably an enteric coating. The coating, in use, prevents degradation of the biopharmaceutical agent in the stomach.

The coating may include the cytochrome P450 3A4 (CYP3A4) inhibitor and/or the P-glycoprotein (P-gp) efflux pump inhibitor.

The transfecting agent may be pCIneo-moLRP::FLAG plasmid.

It is to be understood that other transfecting agents may be used. It is trite that FLAG is particular tag, and could be replaced with other tags if required.

The anti-LRP/LR specific antibody may include igG1-iS18. It is to be understood that igG1-iS18 is commercially available.

LRP/LR may comprise a peptide/protein sequence listing as set forth in SEQ ID NO: 1 or SEQ ID NO: 2, or comprises a fragment thereof as set forth in SEQ ID NO: 4 or SEQ ID NO: 5.

LRP/LR may comprise a peptide/protein sequence listing having at least 80% homology to the sequences as set forth in SEQ ID NO: 1 or SEQ ID NO: 2, or a fragment thereof.

LRP/LR may comprise homologs or fragments thereof, and homologs of the fragments, wherein LRP/LR may comprise a peptide/protein sequence listing as set forth in SEQ ID NO: 1 or SEQ ID NO: 2.

SEQ ID NO: 1 may be a peptide/protein sequence for human LRP/LR and may have the following sequence:

MSGALDVLQMKEEDVLKFLAAGTHLGGTNLDFQMEQYIYKRKSDGIYIINLKRTWEKLLL

AARAIVAIENPADVSVISSRNTGQRAVLKFAAATGATPIAGRFTPGTFTNQIQAAFREPR

LLVVTDPRADHQPLTEASYVNLPTIALCNTDSPLRYVDIAIPCNNKGAHSVGLMWWMLAR

EVLRMRGTISREHPWEVMPDLYFYRDPEEIEKEEQAAAEKAVTKEEFQGEWTAPAPEFTA

TQPEVADWSEGVQVPSVPIQQFPTEDWSAQPATEDWSAAPTAQATEWVGATTDWS

SEQ ID NO: 2 may be a peptide/protein sequence for mouse (*Mus musculus*) LRP/LR and may have the following sequence:

MSGALDVLQMKEEDVLKFLAAGTHLGGTNLDFQMEQYIYKRKSDGIYIINLKRTWEKLLL

AARAIVAIENPADVSVISSRNTGQRAVLKFAAATGATPIAGRFTPGTFTNQIQAAFREPR

LLVVTDPRADHQPLTEASYVNLPTIALCNTDSPLRYVDIAIPCNNKGAHSVGLMWWMLAR

EVLRMRGTISREHPWEVMPDLYFYRDPEEIEKEEQAAAEKAVTKEEFQGEWTAPAPEFTA

AQPEVADWSEGVQVPSVPIQQFPTEDWSAQPATEDWSAAPTAQATEWVGATTEWS

It is to be understood that LRP/LR is highly conserved and homologs or fragments of SEQ ID NO: 1 and SEQ ID NO: 2, and/or homologs of the fragments may also utilized in order to exercise the disclosure described, illustrated and/or exemplified herein.

The peptide/protein sequence of LRP/LR or a homolog or fragment thereof, or a homolog of the fragment, may be bound to, or bonded with, or joined to, or conjugated with, or associated with, an additional protein sequence, amino acid sequence, peptide, protein, or antibody.

Alternatively and/or additionally, the peptide/protein sequence of LRP/LR may form part of a larger and/or longer peptide/protein sequence. In a certain embodiment of the invention LRP/LR may be may be bound to, or bonded with, or joined to, or conjugated with, or associated with, FLAG protein, such that in use, the LRP/LR may be tagged with FLAG. FLAG protein may include a peptide/protein sequence that includes at least a sequence motif DYKDDDDK (SEQ ID NO:3). The Applicant envisages employing other tags.

The fragment of the peptide/protein sequence listing is set forth in SEQ ID NO: 4 corresponding to a fragment of SEQ ID NO: 1 from 102 to 295 and/or SEQ ID NO: 5 corresponding to a fragment of SEQ ID NO: 2 from 102 to 295.

SEQ ID NO: 4 may be a peptide/protein sequence for a fragment of human LRP/LR and may have the following sequence:

RFTPGTFTNQIQAAFREPR

LLVVTDPRADHQPLTEASYVNLPTIALCNTDSPLRYVDIAIPCNNKGAHSVGLMWWMLAR

EVLRMRGTISREHPWEVMPDLYFRDPEEIEKEEQAAAEKAVTKEEFQGEWTAPAPEFTA

TQPEVADWSEGVQVPSVPIQQFPTEDWSAQPATEDWSAAPTAQATEWVGATTDWS

SEQ ID NO: 5 may be a peptide/protein sequence for a fragment of mouse LRP/LR and may have the following sequence:

RFTPGTFTNQIQAAFREPR

LLVVTDPRADHQPLTEASYVNLPTIALCNTDSPLRYVDIAIPCNNKGAHSVGLMWWMLAR

EVLRMRGTISREHPWEVMPDLYFRDPEEIEKEEQAAAEKAVTKEEFQGEWTAPAPEFTA

AQPEVADWSEGVQVPSVPIQQFPTEDWSAQPATEDWSAAPTAQATEWVGATTEWS.

## BRIEF DESCRIPTION

Embodiments of the disclosure will be described below by way of example only and with reference to the accompanying drawings in which:

**FIGURE 1** shows in Example 1 overexpression of LRP::FLAG in THP-1 cells;

**FIGURE 2** shows in Example 1 overexpression of LRP::FLAG significantly increased telomerase activity in both oxLDL treated and untreated THP-1 cells;

**FIGURE 3** shows in Example 1 LRP::FLAG overexpression increases cell viability in oxLDL treated THP-1 cells;

**FIGURE 4** shows in Example 1 extracted DNA that was resolved by agarose gel electrophoresis to determine the effects of LRP::FLAG overexpression on DNA fragmentation;

**FIGURE 5** shows in Example 1 overexpression of LRP::FLAG significantly decreased oxLDL lipid uptake by THP-1 cells;

**FIGURE 6** shows in Example 2 LRP::FLAG overexpression in THP-1 cells using western blotting;

**FIGURE 7** shows in Example 2overexpression of LRP::FLAG significantly increased telomerase activity in THP-1 cells mimicking the diabetic state;

**FIGURE 8** shows in Example 2 the overexpression of LRP::FLAG significantly decreased lipid content in THP-1 cells mimicking type II diabetic state; and

**FIGURE 9** shows in Example 2 LRP::FLAG overexpression induced an increase in glucose uptake in THP-1 cells mimicking the diabetic state.

## DETAILED DESCRIPTION

The content of the Summary above are fully repeated herein by way of reference and to avoid unnecessary repetition. However, non-limiting aspects of the disclosure are provided here to include a biopharmaceutical agent including a 37 kDa/67 kDa laminin receptor precursor/ high affinity laminin receptor (LRP/LR) and/or a fragment thereof for use treatment and/or prevention of atherosclerosis and/or obesity and/or insulin resistance and/or diabetes, wherein LRP/LR and/or the fragment thereof being for administration to a target cell of a subject in need thereof, and wherein the target cell is at least one of the following group: endothelial cells of blood vessels, smooth muscles cells of blood vessels, pancreatic cells including alpha ($\alpha$) cells, beta ($\beta$) cells, delta ($\delta$), and gamma ($\gamma$) cells. In use the biopharmaceutical agent reduces lipid content in the target cell.

An example of an aspect includes a biopharmaceutical agent including a 37 kDa/67 kDa laminin receptor precursor/ high affinity laminin receptor (LRP/LR) and/or a fragment thereof for use treatment and/or prevention of atherosclerosis, wherein LRP/LR and/or the fragment thereof being for administration to a target cell of a subject in need thereof, and wherein the target cell is at least one of the following group: endothelial cells of blood vessels and smooth muscles cells of blood vessels. In use the biopharmaceutical agent reduces lipid content in the target cell.

An another example of an aspect includes a biopharmaceutical agent including a 37 kDa/67 kDa laminin receptor precursor/ high affinity laminin receptor (LRP/LR) and/or a fragment thereof for use treatment and/or prevention of obesity and/or insulin resistance and/or diabetes, wherein LRP/LR and/or the fragment thereof being for administration to a target cell of a subject in need thereof, and wherein the target cell is at least one of the following group: pancreatic cells including alpha ($\alpha$) cells, beta ($\beta$) cells, delta ($\delta$), and gamma ($\gamma$) cells. In use the biopharmaceutical agent reduces lipid content in the target cell.

The LRP/LR is typically encapsulated into a delivery means comprising nanoparticles, preferably the nanoparticles being functionalized with chemical, biochemical or biological moieties to ensure site specific delivery to the target cell, wherein the moieties act as ligands to ensure site specific delivery at the target cell The delivery means is typically formulated into a pharmaceutical composition, which pharmaceutical composition including a pharmaceutical carrier for parenteral or non-parenteral administration to the subject.

The pharmaceutical composition may further include an active pharmaceutical ingredient (API). The API may treat atherosclerosis and/or obesity and/or insulin resistance and/or diabetes, and/or co-morbid conditions thereof.

The pharmaceutical composition may further include an anti-oxidant such that in use at the target cell the anti-oxidant scavenges reactive oxygen species.

The LRP/LR typically comprises a peptide/protein sequence listing as set forth in SEQ ID NO: 1 or SEQ ID NO: 2, or comprises a fragment thereof as set forth in SEQ ID NO: 4 or SEQ ID NO: 5.

The LRP/LR may form part of a transfecting agent for expressing a 37 kDa/67 kDa laminin receptor precursor/ high affinity laminin receptor (LRP/LR) and/or a fragment thereof, preferably the transfecting agent is pCIneo-moLRP::FLAG plasmid. In other words, a transfecting agent may be used to upregulate LRP/LR.

The nanoparticles are typically polymeric nanoparticles and biodegradable to provide in use a reduced risk of an immunogenic response to said polymeric nanoparticles, and further wherein the polymeric nanoparticles are biocompatible to mitigate risk of any immunogenic response to said polymeric nanoparticles, and further wherein said polymeric nanoparticles are one or more selected from the following group: eudragit, gum arabic, carrageenan, cellulose, hydroxypropyl cellulose (HPC), methylcellulose (MC), hydroxypropylmethylcellulose (HPMC), polylactic-co-glycolic acid (PLGA), chitin, pectin, amylopectic, natural rubber, polyethylene, polypropylene, polystyrene, polyamide, polyacrylonitrile, polyvinyl chloride, polyvinyl alcohol (PVA), polyethylene glycol (PEG), polyethylene oxide (PEG), poly(D-lactide) (PDLA), polylactic acid (PLLA), polygalacturonate, methylcellulose (polyacetals), poly($\varepsilon$-caprolactone), phospholipids, polysaccharides, polyanionic polysaccharides, carboxymethyl cellulose, carboxymethyl amylose, chondroitin-6-sulfate, dermatin sulfate, heparin, heparin sulfate, poly(hydroxyethyl methylacrylate), collagen, fibrinogen, albumin, fibrin, acrylamide, hydroxypropyl methacrylamide-based copolymers, polyacrylamide, poly(N-isopropyl acrylamide) (pNIPAAm), polyvinylpyrrolidone, poly(methacrylic acid-g-ethylene glycol), poly(N-2-hydroxypropyl methacrylamide), poly(glycolic acid) (PGA), poly(lactic acid) (PLA), chitosan, poly(2-hydroxyethylmethacrylate) (HEMA), polyphazene, phosphorylcholine, hyaluronic acid (HA), hydroxyethyl methacrylate (HEMA), methylene-bis-acrylamide (MBAAm), poly(acrylic acid) (PAAc), poly-acrylamide (PAAm), polyacrylonitrile (PAN), polybutylene oxide (PBO), polycaprolactone (PCL), polyethylene imine) (PEI), poly(ethyl methacrylate) (PEMA), propylene fumarate (PF), poly(glucosylethyl methacrylate) (PGEMA), poly(hydroxy butyrate) (PHB), poly(hydroxyethyl methacrylate) (PHEMA), poly(hydroxypropyl methacrylamide) (PHPMA), poly(methyl methacrylate) (PMMA), poly(N-vinyl pyrrolidone) (PNVP), polypropylene oxide) (PPO), poly(vinyl acetate) (PVAc), poly(vinyl amine), chondroitin sulfate, dextran sulfate, polylysine, gelatin, carboxymethyl chitin, dextran, agarose, pullulan, polyesters, PEG-PLA-PEG, PEG-PLGA-PEG, PEG-PCL-PEG, PLA-PEG-PLA, poly(PF-co-EG), poly(PEG/PBO-terephthalate), PEG-bis-(PLA-acrylate), PEG6CDs, PEG-g-poly(AAm-co-vinlyamine), poly(NIPAAm-co-AAc), poly(NIPAAm-co-EMA), PNVP, poly(MMA-co-HEMA), poly(AN-co-allyl sulfonate), poly(biscarboxy-phenoxy-phosphazene), poly(GEMA-sulfate), poly(PEG-co-peptides), alginate-g-(PEO-PPO-PEO), poly(PLGA-co-serine), collagen-acrylate, alginate, alginate-acrylate, poly(HPMA-g-peptide), HA-g-NIPAAm, and poly(vinyl methyl ether) (PVME), and/or derivatives of any one or more of the aforementioned..

Typically, the delivery means is formulated for oral delivery and includes at least partially thereabout a coating including an inhibitor of cytochrome P450 3A4 (CYP3A4) selected from the group consisting of polyethylene glycol, polyamine, polymethyl methacrylate and derivatives thereof, and wherein the coating further includes a P-glycoprotein (P-gp) efflux pump inhibitor.

The transfecting agent and/or the LRP/LR and/or the fragment of LRP/LR substantially as herein described are all examples of biopharmaceutical agents employed as part of this disclosure. It is to be understood that LRP/LR is highly conserved and homologs or fragments of SEQ ID NO: 1 and SEQ ID NO: 2, and/or homologs of the fragments may also utilized in order to exercise the disclosure described, illustrated and/or exemplified herein.

Substantially identical sequences may also be employed. As used herein, a substantially identical sequence is an amino acid or nucleotide sequence that differs from a reference sequence only by one or more conservative substitutions, or by one or more non-conservative substitutions, deletions or insertions located at positions of the sequence that do not destroy or substantially reduce the activity of one or more of the expressed polypeptides or of the polypeptides encoded by the nucleic acid molecules.

In the examples below, the transfecting agent is be pCIneo-moLRP::FLAG plasmid. It is to be understood that another transfecting agent for use in the upregulation of LRP/LR expression in the target cells is envisaged by the Applicant. Detailed example embodiments of the disclosure, which are not limiting to the scope of the disclosure, are provided herein below as Examples 1 and 2.

## EXAMPLES

## EXAMPLE 1 - atherosclerosis

## Materials and methods

## Cell culture for THP-1 (human leukemic monocyte) cells

The human monocyte cell line (THP-1) was used for all experimental procedures carried out. This cell line was chosen for its ability to readily take up oxLDL (oxidised low density lipoprotein) and become foam cells, which is the major component of atherosclerotic plaques. RPMI media with 15% foetal bovine serum (FBS), 1% MEM non-essential amino acids was used to provide the essential nutrients to culture both the un-transfected and transfected THP-1 cells. In addition, 2% penicillin/streptomycin was used in an attempt to prevent bacterial contamination. THP-1 cells grow both adherently as well as in suspension, and thus require extra media in order to grow; therefore these cells were cultured in a 5 ml flask using 15 ml of media. The cells were stored in an incubator pre-set to 37 °C with 5% $CO_2$ in order to mimic the *in vivo* conditions present within the human body. Media changes were performed two to three times a week. Additionally, when flasks reached a confluency of approximately 70-80%, a 1:10 split was performed.

For media changes and cell passaging, the flasks were gently scraped using a cell scraper and the media was placed into centrifuge tubes and centrifuged for 10 minutes at 4000 rpm. The old media was discarded, and the pellet was re-suspended in 2 ml of Dulbecco's phosphate buffered saline (D-PBS), in order to remove debris, and centrifuged at 4000 rpm for another 10 minutes. The pellet was then re-suspended in 1 ml of media, and if the confluency was below 70% the entire 1 ml was then put back into the flask. In confluent flasks, cells were split in a ratio of 1: 10. The remainder of the cells were pelleted and re-suspended in 90% FBS with 10% DMSO, and frozen at -80 °C as stocks.

## Overexpressing LRP::FLAG and treating THP-1 cells

In order to overexpress LRP::FLAG, the THP-1 cells were transfected with the pCIneo-moLRP::FLAG plasmid using the Xfect transfection kit and protocol. Flasks that were at approximately 70% confluency were chosen for transfection. Briefly, Xfect reaction buffer was added to 5 μg of the pCIneo-moLRP::FLAG plasmid in order to make 100 μl of sample. Thereafter 1.5 μl of Xfect polymer was added to the mixture and the reagents were incubated at room temperature for 10 minutes in order to allow the nanoparticle complexes to form. The reaction mix was then drop-wise added to the flask. The cells were treated with 10 μl of geneticin (50 mg/ml) once every 3 weeks in order to promote transcription of the plasmid and ensure transfection had occurred.

In order to induce atherosclerotic plaque formation, the THP-1 cells were treated with oxLDL (Bio-Rad). Briefly, un-transfected and transfected cells were seeded into 6, 48 or 96 well plates and allowed to recover and grow for 24-48 hours. When the cells reached 70-80% confluency, a treatment consisting of 50 μg/μl oxLDL was added to each well of the respective plates and cells were incubated for 48 hours. Thereafter, the cells from each well were harvested as pellets or experiments were performed in the wells. For each experiment the following treatments were used: untreated un-transfected, untreated transfected, treated un-transfected and treated transfected.

Bicinchoninic acid™ (BCA) protein assay

The BCA assay was performed in order to obtain the concentration of protein extracted from each cell treatment and ensure equal loading for western blotting. For this technique, cells from flasks that were approximately 70% confluent were scraped and centrifuged at 10000 rpm for 10 minutes. The pellets were then resuspended in RIPA lysis buffer (consisting of 50 mM Tris-HCl at pH 7.4, mixed with 50 mM NaCl, 2 mM EDTA and 0.1% SDS). The SDS in the buffer linearizes the extracted proteins and provides a negative charge proportional to the molecular mass of the protein. Next, 5 μl of each lysate, together with 20 μl of distilled water, was loaded into a 96 well plate. Additionally, 25 μl of a set of standards (0 mg/ml, 0.2 mg/ml, 0.4 mg/ml, 0.6 mg/ml, 0.8 mg/ml and 1 mg/ml), made up of serial dilutions (1:10) of Bovine Serum Albumin (BSA), was loaded. This was done in order to construct a standard curve, which was utilized to determine the protein concentration of the unknown lysates. Thereafter, 200 μl of a Bicinchoninic acid and copper sulphate (930 μl: 30 μl) mix was added to each well, and the plate was incubated at 37 °C for 30 minutes. This allowed time for the peptide bonds to reduce $Cu^{2+}$ to $Cu^+$ in order to produce a colour change proportional to the amount of protein present. The Sunrise Teccan ELISA plate reader was used to measure the colour change at 562 nm and the concentration of the samples was obtained from the standard curve.

Western blotting & SDS-PAGE

SDS PAGE and western blotting is used for immunological detection and quantification of proteins. It is a well-established technique that is simple to use and is particularly useful in this study as it allows for the detection of LRP::FLAG in order to confirm transfection. In addition, it is useful for detecting changes in CVD markers in cells overexpressing LRP::FLAG, and for this reason it was chosen above other protein detection techniques. The shortfalls of this technique include the potential for non-specific binding of antibodies, the length of the procedure and that it may be affected by contaminants. The technique makes use of sodium dodecyl sulphate polyacrylamide gel electrophoresis (SDS-PAGE), followed by blotting onto a PVDF membrane and detection with a specific antibody complex targeting the protein of interest. The LRP::FLAG and β-actin loading control, which was extracted from the THP-1 cells, were diluted to a final concentration of 100 μl/ml. This allowed for equal and accurate separation of protein during SDS-PAGE.

SDS-PAGE

SDS-PAGE was performed in order to linearize and separate the different proteins based on their molecular mass. The SDS from the RIPA buffer binds proportionally to the size of the proteins, giving them an equal charge to mass ratio and allowing them to be separated solely based on their molecular size. This allows for accurate detection of proteins with known molecular weights by using a molecular weight marker. A 10% polyacrylamide gel was prepared in two parts. Firstly, the separating gel was made by mixing together 4.8 ml of distilled water, 2.5 ml of 40% acrylamide, 2.5 ml of separating buffer (187 g of Tris base, pH 8.8, and 0.4% SDS in 1 litre of distilled water), 100 μl of 10% SDS, and 100 μl of 3% APS and 5 μl of TEMED. This was then added to the plates and allowed to polymerize. Thereafter, the stacking gel was prepared by adding 3.65 ml of distilled water to 625 μl of 40% acrylamide, 625 μl stacking buffer (60.5 g of Tris base, pH 6.8, and 4% SDS in 1 litre of distilled water), 50 μl of 10% SDS, and 50 μl of APS and 5 μl of TEMED. This was added on top of the separating gel, a comb was inserted and the gel was allowed to polymerize. The stacking gel allowed the proteins to accumulate in a single band at the bottom of the well, while the separating gel allowed for the separation of the different proteins as individual bands across the gel. The lysate from the extraction was then mixed with loading dye and the samples were incubated at 95 °C for 5 minutes, in order to allow the dye to intercalate with the proteins. Thereafter, 5 μl of each sample and a molecular weight marker was loaded in to the wells. The proteins were separated at 120 V in running buffer until the dye front was approximately 1 cm from the bottom of the gel (approximately 45 minutes).

Western blotting

After the proteins were separated they were blotted onto a PVDF membrane, where they could be incubated with the appropriate antibodies (table 5.4.1). First, the membrane was placed in methanol (to activate) and then, together with blotting paper, it was placed in transfer buffer (to equilibrate). Thereafter, the membrane, with the gel positioned on top, was placed in between the blotting paper, which was placed in the semi-dry transfer blotter apparatus, and electroblotting was performed at 120 V for 45 minutes. Once the transfer was complete, the PVDF membrane was blocked with 10 ml of 3% BSA in order to prevent non-specific antibody binding. The membrane was then incubated with the appropriate primary antibody (table 5.4.1) at 4 °C overnight. Thereafter, five washes with PBS-tween were performed for 5 minutes each. The membrane was incubated with the appropriate secondary antibody, if applicable (table 5.4.1), and for one hour at room temperature. This was followed by a further five washes. Next, the membrane was incubated with 4 ml of Clarity Western ECL substrate (Bio-Rad) in the dark for 1 minute and then viewed with the ChemiDock imaging system (Bio-Rad) where images were taken. Densitometric analysis allows for the quantification of the protein levels. However the late delivery of oxLDL treatment, together with constant contamination, resulted in an insufficient amount of time to perform the densometric analysis and quantify the protein extracted.

**Table 5.4.1: A list of the primary and secondary antibodies used in the western blotting protocol.**

| Target protein | Primary antibody | Secondary antibody | Dilution factor for both antibodies. |
|---|---|---|---|
| LRP::FLAG | Murine anti-FLAG (Sigma-Aldrich®) | Anti-murine-IgG-HRP (Sigma-Aldrich®) | 1:4000 |
| β-actin | Murine-anti-β-actin-peroxidase (Sigma-Aldrich®) | None | 1:10000 |

### Detecting telomerase activity

Shortened telomeres are a hallmark of CVD, and are largely caused due to decreased activity of the telomerase enzyme. qPCR was used to assess the effect of LRP::FLAG overexpression on telomerase activity in the differentially treated cells. The TRAPeze RT® telomerase detection kit (Merck Millipore™) was used to extract active telomerase and allow for telomeric substrate extension in order to provide a relative indication of telomerase activity. This technique is very accurate in determining telomerase activity as it extends substrates relative to the amount of protein available, and for this reason the technique was chosen. Cells were initially seeded into 6 well plates and treated with oxLDL. After 48 hours, the wells were pelleted and resuspended in 200 µl of Chaps lysis buffer. The sample was then incubated for 30 minutes at 4 °C in order to extract the protein. Thereafter, the sample was centrifuged at 12000 rpm for 20 minutes at 4 °C, and the protein concentration was determined using the Nanodrop ND-1000 spectrophotometer (Thermo Scientific). The protein samples were then diluted down to 500 ng/µl, and 30 µl of protein samples were incubated for 20 minutes at 100 °C in order to provide a heat treated negative control in which telomerase is deactivated. A control and reaction mix was made using PCR grade water, Taq polymerase and control or reaction mix. In a 96 well plate, the control mix was added to the heat treated samples and the chaps negative control, while the reaction mix was added to all protein samples, the standards and the positive control. The plate was then covered with a cover slip and placed in the Arya qPCR machine. The following parameters were used: an initial 10 minute denaturation step at 95 °C, then 45 amplification cycles, each consisting of: 10 seconds for denaturation at 95 °C, 10 seconds for annealing at 58 °C and 60 seconds for extension at 72 °C. The data was normalized to the standard curve and analysed using student's *t*-test.

### Cell counting

Determining the number of cells is required in order to seed an equal amount of cells for assays, such as the MTT assay and oil red stain, in order to obtain accurate data. A haemocytometer was used to determine the amount of cells required for seeding. Briefly, cells were scraped and 10 µl of cell suspension was mixed with 10 µl of filtered trypan blue. Cells were then counted with a haemocytometer and the cell concentration was determined using the equation:

$$Cell\ concentration\left(\frac{cells}{ml}\right) = cells\ per\ 16\ squares\ x\ 10^4\ x\ dilution\ factor$$

The cell concentration was then used to seed an equal concentration of cells into each well of the plates used in order to ensure that the data obtained is accurate.

### Detecting changes in cell viability in cells overexpressing LRP::FLAG

The MTT cell viability assay is a well established technique that is used to determine cell viability. This assay makes use of MTT, (3-(4, 5-Dimethylthiazol-2-yl)-2, 5-diphenyltetrazolium bromide), a chemical that is converted to the insoluble formazan in the mitochondria of live cells (van Meerloo et al., 2011). Cells treated with oxLDL undergo apoptosis as they become foam cells (Wintergerst et al., 2000), thus detecting cell viability can determine if overexpressing LRP::FLAG rescues cells from apoptosis. For this assay, 8000 cells were seeded into a 48 well plate, treated with 50

$\mu$g/$\mu$l oxLDL and incubated for 48 hours. Thereafter, 7 mg of MTT (Duchefa Biochemie) was dissolved into 1.5 ml of PBS and then filter sterilized. Next, 100 $\mu$l of the 0.5 mg/ml MTT solution was added to each well and the plate was incubated at 37 °C for approximately 3 hours. Finally, the formazan crystals that formed were dissolved in 250 $\mu$l DMSO and the colour changes were detected at 590 nm using the Sunrise Teccan ELISA plate reader. The data obtained was analysed using student's t-test and then plotted onto a histogram.

### Assessing the lipid content in cells overexpressing LRP::FLAG

In treated cells, the uptake of oxLDL results in an increase in lipid content in the cells due to the lipid moiety of the lipoprotein (Grahame and Schlesinger, 2012). Additionally, oxLDL promotes the uptake of cholesterol and triacylglycerol into cells (Batt et al., 2004). Therefore, assessing the lipid content in the cells provides possible insight on how the overexpression of LRP::FLAG affects oxLDL uptake, and subsequently atherosclerotic plaque formation. The Oil Red O lipid staining kit from Sigma-Aldrich® stains the lipids in the cells, allowing for a quantitative measure of oxLDL present in the cells. This technique is a quick and simple technique that can accurately determine lipid content. In addition, the stain was extracted in order to quantify the amount of lipid present, giving a more accurate indication of the effects of LRP::FLAG overexpression. The shortfall to this technique is that the large number of required washes may remove some of the cells, resulting in skewed data. This technique was chosen above others as it provides both qualitative and quantitative data. Once the number of cells was obtained using a haemocytometer, 16000 transfected and untransfected cells were seeded into a 96 well plate, with the appropriate wells being treated with oxLDL and incubated for 48 hours. Thereafter, the cells were washed with 100 $\mu$l of PBS twice, and then incubated with 10% formalin at room temperature for 45 minutes in order to fix them. After the incubation period, the cells were washed twice with distilled water followed by 5 minute incubation with 60% isopropanol. Next, a working oil red solution of 3 parts stock and 2 parts distilled water was prepared and 200 $\mu$l was added to each well and incubated for 15 minutes. The cells were washed 3 times with water and images were taken with an inverted light microscope. Additionally, the stain was extracted using 100% isopropanol and quantified at 490 nm in order to accurately determine the changes in lipid content. The data obtained was analysed via student's *t*-test and plotted onto a histogram.

### Statistical analysis

All data obtained during experimentation was subjected to critical statistical analysis, using Microsoft Excel 2010, in order to ensure that the data was relevant and significant. In addition, each experiment was done in at least triplicates in order to ensure accuracy of the data, as well as to calculate standard deviation. The two-tailed student's t-test was used for analysis at a 95% confidence interval. Values where *p<0.05 was considered statistically significant, while values where **p<0.01 and ***p<0.001 were considered as highly significant.

### RESULTS

### Confirmation of LRP::FLAG overexpression in transfected THP-1 cells.

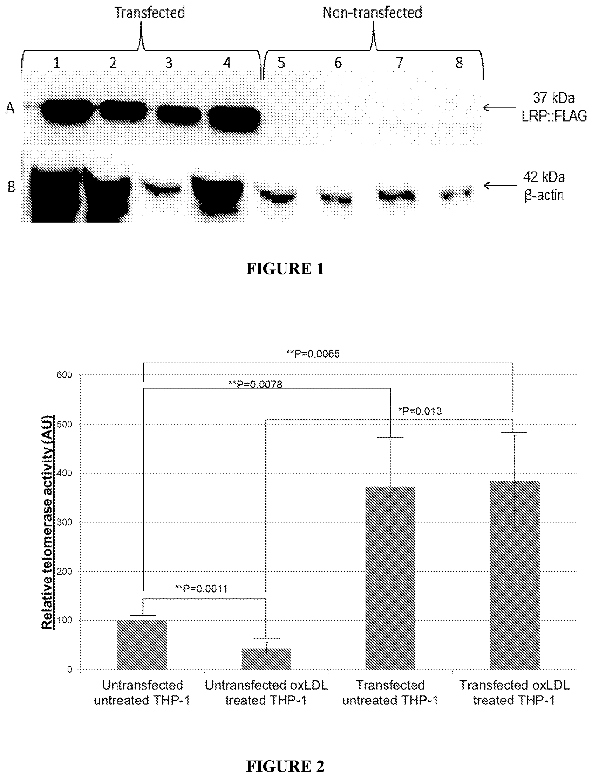

To determine if LRP/LR has a potential role in CVD, THP-1 cells were stably transfected using the pCIneo-moLRP::FLAG plasmid in order to overexpress LRP::FLAG in the THP-1 cells. After transfection, western blotting was performed in order to confirm the overexpression of LRP::FLAG (Figure 1). $\beta$-actin (loading control) was present in both untransfected and transfected THP-1 cells (lanes 1-8), while LRP::FLAG was present only in the transfected cells (lanes 5-8), indicating that the transfection was successful and that LRP::FLAG was overexpressed. Overexpression of LRP::FLAG by the pCIneo-LRP::FLAG plasmid was assessed in transfected (lanes 1-4) and untransfected (lanes 5-8) THP-1 cells. LRP::FLAG was detected using the murine anti-FLAG primary antibody and the murine anti-IgG-HRP secondary antibody, while $\beta$-actin was detected using the murine anti-actin-peroxidase antibody.

### Overexpression of LRP::FLAG significantly increases telomerase activity in transfected THP-1 cells treated with oxLDL

A key factor in CVD is critically shortened telomeres, which occurs due to impediment of telomerase activity by high levels of oxidative stress. To determine if the overexpression of LRP::FLAG subsequently affected telomerase activity, qPCR was performed on transfected and untransfected THP-1 cells that were treated with oxLDL. A significant 57.43% decrease in telomerase activity was observed in untransfected treated cells relative to untreated untransfected THP-1 cells. Furthermore, a 272.72% and 283.47% increase in telomerase activity was observed in transfected untreated and treated cells respectively when compared to the untransfected and untreated THP-1 cells (Figure 2).

The graph depicts the changes in relative telomerase activity after LRP::FLAG overexpression. The data was normalized in order to plot the graph. A significant 57.43% decrease in telomerase activity was found in untransfected cells (blue) when treated with oxLDL (**p<0.01), while the telomerase activity was significantly increased in cells overexpressing LRP::FLAG (red) irrespective of oxLDL treatment (**p<0.01). Transfection of cells significantly increased telomerase activity by 3.8 fold, indicating that LRP::FLAG may play a role in maintaining telomerase activity in a CVD environment.

Overexpression of LRP::FLAG significantly increases cell viability in THP-1 cells treated with oxLDL

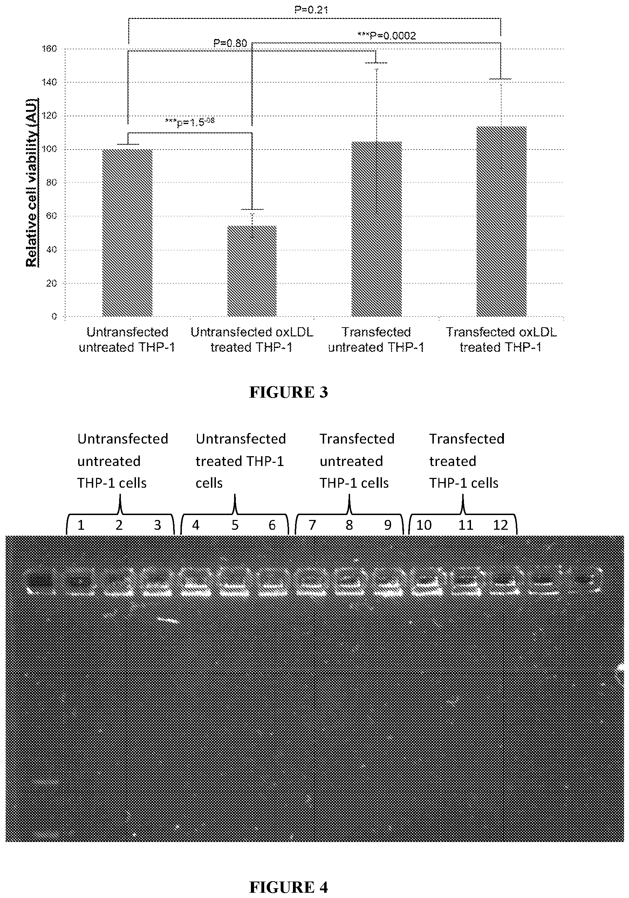

One of the hallmarks of oxLDL uptake in cells is the induction of apoptosis and thus decreased cell viability. Cells were treated with oxLDL in order to mimic an atherosclerotic environment and to study the effect overexpressing LRP::FLAG has in CVD. In order to assess the effect that overexpressing LRP::FLAG has on cell viability, the MTT cell viability assay was performed using both transfected and untransfected THP-1 cells. A significant 45.53% decrease in cell viability was found between untreated and oxLDL treated untransfected THP-1 cells. When the cells were transfected, cell viability remained at the approximate normal (untransfected untreated) level regardless of treatment (Figure 3). The graph depicts the change in cell viability when cells are treated and/or transfected. Untransfected cells (blue) treated with oxLDL show a significant 1.8 fold decrease in cell viability (***p<0.001). No significant decrease in cell viability was observed after oxLDL treatment when cells were transfected (red), indicating that LRP::FLAG rescues cells from oxLDL induced cytotoxicity.

**The effect of overexpression of LRP::FLAG on DNA fragmentation**

The formation of atherosclerotic plaques occurs when monocytes become foam cells through apoptosis. During apoptosis DNA is fragmented and fragmented DNA results in a smear when resolved by agarose gel electrophoresis. Therefore, detecting changes in DNA fragmentation through agarose gel electrophoresis was used to assess the role LRP::FLAG overexpression plays in DNA fragmentation. However, the DNA remained trapped within the wells of the gel (Figure 4). A reduction in smearing in the transfected treated cells when compared to the untransfected treated cells was expected, however due to time limitations this experiment could not be repeated.

The DNA extracted from the untransfected untreated (lanes 2-4), untransfected oxLDL treated (lanes 5-7), transfected untreated (lanes 8-10) and transfected oxLDL treated (lanes 11-13) were all trapped in the wells. This may have occurred due to the genomic DNA fragments being too large for the gel, resulting in the DNA remaining in the wells. The use of a restriction enzyme may have solved this problem. Furthermore, this technique has a low sensitivity, thus any fragments that did leave the well may have not been detected due to the low concentration of DNA extracted.

**Overexpression of LRP::FLAG significantly reduces the lipid content in THP-1 cells treated with oxLDL**

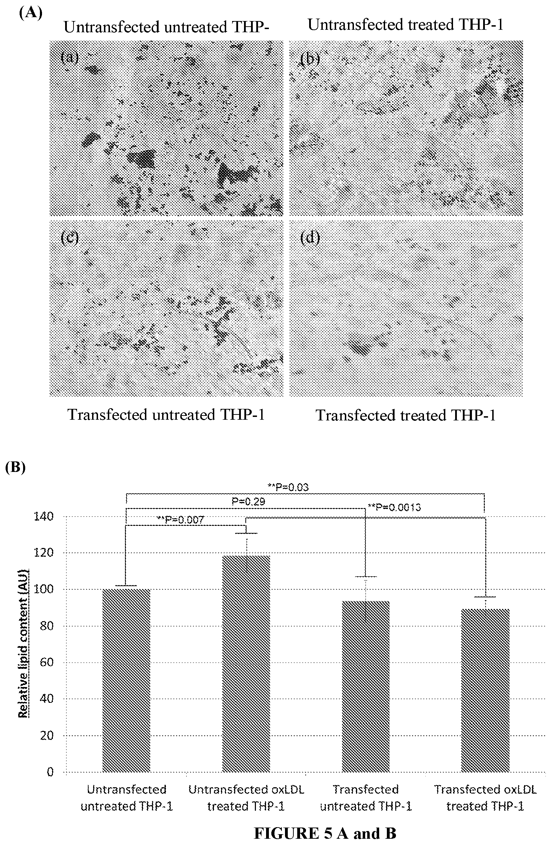

The formation of atherosclerotic plaques requires monocytes to take up oxLDL, which results in an increased cell lipid content. This major hallmark was investigated, using the oil red lipid staining assay, in order to assess whether or not LRP::FLAG overexpression plays a role in atherosclerotic plaque formation. The assay showed that treatment with oxLDL resulted in an increase in lipid content (red staining) in the untransfected cells when compared to the untreated untransfected cells (Figure 5 A a-b). Furthermore, transfection resulted in decreased lipid content in both THP-1 cells treated with oxLDL and cells without treatment, as compared to the untransfected and untreated cells (Figure 5 A a, c and d). In addition, quantification of the stains showed a significant increase in lipid content between untreated and treated untransfected THP-1 cells. A significant decrease in lipid content was observed between the untransfected treated and transfected treated cells, while the transfected untreated cells returned to the normal (untreated untransfected THP-1 cells) lipid level (Figure 5 B).

Panel A qualitatively shows the differences observed when THP-1 cells were treated and/or transfected. The images chosen are representative of the stains present in all 6 wells. The images show an increase in red staining in untransfected cells which are treated with oxLDL (b) as compared to untransfected and untreated cells (a). Cells that are transfected show a decrease in oil red stain regardless of treatment (c and d). Panel B shows a quantitative representation of the stained cells. Untransfected cells (blue) treated with oxLDL had a significant increase in oxLDL lipid concentration within the cells (**p<0.01). Transfection resulted in a significant decrease (**p<0.01) in the lipid content in the cells, bringing the concentration back to the untransfected and untreated cell level (red).

**Discussion**

It is known that telomere length is significantly reduced in monocytes prone to oxLDL uptake and it has been shown that these monocytes have a reduction in telomerase (Fitzpatrick et al., 2007). It has also been shown that telomerase activity is upregulated, with a subsequent increase in telomere length, once plaques have been formed but with no observable effect on the plaques (Huzen et al., 2011). Consequently, an upregulation of LRP/LR is not expected to have any effect on plaques. It is therefore surprising and unexpected that upregulating telomerase activity through use of LRP::FLAG causes a reduction in atherosclerotic plaque formation and rescue cells from oxLDL induced apoptosis.

**Overexpression of LRP::FLAG in THP-1 cells.**

Human leukemic monocyte (THP-1) cells were used for its ability to readily take up oxLDL and become foam cells. To assess the function of LRP::FLAG in these cells, they were transfected using the pCIneo-moLRP::FLAG construct and transfection was confirmed by western blotting. The western blot showed that LRP::FLAG was expressed in the transfected THP-1 cells and not the untransfected cells (Figure 1).

**The effect of LRP::FLAG on telomerase activity**

Upon confirmation of transfection, the effect of LRP::FLAG overexpression on telomerase activity in a cardiovascular cell (atherosclerotic) culture model was assessed.

Telomerase activity in monocytes is low due to the fact that these cells have a low turnover (Demissie et al., 2006). As a result, telomeres are damaged and eroded when oxLDL is taken up by these cells. In addition, oxLDL decreases telomerase activity due to induction of an oxidative state in the cells, resulting in recruitment of the hTERT subunit to the mitochondria (Cong et al., 2002). This prevents it from functioning in the telomerase enzyme which is situated in the nucleus. qPCR analysis was used in order to assess how the overexpression of LRP::FLAG influenced telomerase activity. The results confirmed that the relative telomerase activity was upregulated in the transfected THP-1 cells (Figure 2). In fact, when the untransfected THP-1 cells were treated with oxLDL, a significant reduction in the relative telomerase activity was seen (Figure 2).

These studies show that oxLDL decreases telomerase activity in a concentration dependent manner. Without being limited to theory this may be through the inactivation of PI3K/Akt via dephosphorylation. Interestingly, it was further shown that when the THP-1 cells were transfected, the telomerase activity increased drastically regardless of treatment. Without being limited to theory, this is likely due to LRP::FLAG inducing an increase in hTERT expression. Since hTERT is the limiting factor in telomerase, the increased concentration of hTERT may have been responsible for the increase in telomerase activity. However, since hTERT has extra telomeric effects, the increase in its concentration is likely not the only cause for the increased telomerase activity (Cong et al., 2002).

It was found that the increase in telomerase activity was enough to reverse the effects of oxLDL treatment. Furthermore, it was observed that telomerase activity was increased by 3.8 fold between the untransfected untreated cells and the transfected treated cells (Figure 2), and was evident in both untreated and treated transfected THP-1 cells. The observed upregulation of telomerase activity would likely result in an increase in telomere length, which counteracts the observed shortened telomere length associated with atherosclerosis. This causes a reduction in plaque formation and/or reversing and/or preventing atherosclerosis.

**The effect of LRP::FLAG on cell viability**

In order to determine the effects of upregulation of telomerase activity in atherosclerosis, cell viability was assessed using the MTT assay. The oxLDL treated untransfected THP-1 cells had a significant 1.8 fold decrease in cell viability (Figure 3). This is consistent with literature, which has shown that oxLDL induces apoptosis by rapid activation of the caspase-3 apoptotic pathway (Wintergerst et al., 2000).

In transfected untreated cells, the cell viability remained similar to the untransfected untreated cells, indicating that transfection does not cause overproliferation. This is an indication that LRP::FLAG overexpression does not cause cells to become tumorigenic which is surprising. Similarly, there is no significant change in cell viability between the untransfected untreated cells and transfected treated cells, indicating that transfection rescues cells from oxLDL induced apoptosis. Since monocytes undergo apoptosis to become foam cells, rescuing them from apoptosis is essential to preventing atherosclerotic plaque formation. The increase in cell viability is likely due to the increased telomerase activity previously shown, which causes telomere extension.

Interestingly, a slight increase in cell viability of the transfected treated cells was detected (Figure 3). Though insignificant, this increase is likely due to oxLDL inducing overproliferation in cells. Without being limited to theory, it is shown that transfection increases cell viability in oxLDL treated cells likely by rescuing cells from oxLDL induced apoptosis. This suggests that LRP::FLAG has a role in preventing/reducing the effects of atherosclerosis in a cardiovascular (particularly a atherosclerotic) cell culture model.

**The effect of LRP::FLAG on lipid content.**

A major hallmark of atherosclerosis is the increased lipid content in cells. This occurs when the lipid content of oxLDL is taken up by cells, by upregulation of LOX-1 and ACAT1,2, increasing cellular cholesterol and triacylglycerol content (Batt et al., 2004). To further substantiate that the overexpression of LRP::FLAG and the subsequent increase in telomerase activity plays a role in atherosclerosis or cardiovascular disease, cellular lipid content was assessed.

In order to assess this, the oil red lipid stain was used to obtain a qualitative, as well as a quantitative measure of the lipid content (Figure 5). Since lipid content is increased in atherosclerotic plaques, a decrease in lipid content provide evidence that transfection aids in the reversal and/or prevention of atherosclerotic plaque formation. It was found that treatment with oxLDL did cause increased lipid content in the untransfected THP-1 cells. This corresponds with literature which showed that oxLDL uptake causes increased lipid content due to the lipid component of oxLDL, as well as the subsequent lipid uptake it causes (Batt et al., 2004). Transfected cells when treated with oxLDL, showed a reduction in the lipid content which was significantly lower (11%) than untransfected treated cells. A possible reason for this is that LRP::FLAG may play a role in increasing lipid metabolism in the cells.

**Conclusion**

In conclusion, it was shown that overexpressing LRP::FLAG increased telomerase activity in a THP-1 atherosclerotic cell culture model. Additionally, the overexpression of LRP::FLAG increased the viability of cells that were treated with oxLDL. Furthermore, transfection was shown to rescue oxLDL treated cells from the increased lipid content which is characteristically seen in atherosclerotic plaques. The oil red stain assay revealed that overexpression of LRP::FLAG in cells treated with or without oxLDL caused a significant decrease in cellular lipid content, which is usually increased in atherosclerosis. These findings show that LRP/LR likely plays a role in atherosclerosis, and that overexpressing LRP::FLAG may offer a potential novel therapeutic treatment to reduce or impede the formation of atherosclerotic plaques.

Increasing telomere length after plaque formation has been shown to have no effect on the plaques themselves. Consequently, upregulation of LRP/LR was not expected to have any effect on the plaques and/or their formation. It is therefore unexpected and surprising that upregulating telomerase activity by providing LRP/LR to a cell and/or the upregulation of LRP/LR causes a decrease in lipid content and/or rescues cells from oxLDL induced apoptosis.

**REFERENCES**

AUBERT, G. & LANSDORP, P. M. 2008. Telomeres and aging. Physiological reviews, 88, 557-579. BENTZON, J. F., OTSUKA, F., VIRMANI, R. & FALK, E. 2014. Mechanisms of plaque formation and rupture. Circulation research, 114, 1852-1866.

BLACKBURN, E. H., GREIDER, C. W. & SZOSTAK, J. W. 2006. Telomeres and telomerase: the path from maize, Tetrahymena and yeast to human cancer and aging. Nature medicine, 12, 1133-1138.

BLASCO, M.A., 2005. Mice with bad ends: mouse models for the study of telomeres and telomerase in cancer and aging. The EMBO journal, 24(6), pp.1095-1103.

BREITSCHOPF, K., ZEIHER, A.M. AND DIMMELER, S., 2001. Pro-atherogenic factors induce telomerase inactivation in endothelial cells through an Akt-dependent mechanism. FEBS letters, 493(1), pp.21-25.

CHEN, Q., VAZQUEZ, E. J., MOGHADDAS, S., HOPPEL, C. L. & LESNEFSKY, E. J. 2003. Production of reactive oxygen species by mitochondria central role of complex III. Journal of Biological Chemistry, 278, 36027-36031.

CONG, Y.-S., WRIGHT, W. E. & SHAY, J. W. 2002. Human telomerase and its regulation. Microbiology and molecular biology reviews, 66, 407-425.

CONTE, M.S., POMPOSELLI, F.B., CLAIR, D.G., GERAGHTY, P.J., MCKINSEY, J.F., MILLS, J.L., MONETA, G.L., MURAD, M.H., POWELL, R.J., REED, A.B. AND SCHANZER, A., 2015. Society for Vascular Surgery practice guidelines for atherosclerotic occlusive disease of the lower extremities: management of asymptomatic disease and claudication. Journal of vascular surgery, 61(3), pp.2S-41S.

COUNTER, C. M., GUPTA, J., HARLEY, C. B., LEBER, B. & BACCHETTI, S. 1995. Telomerase activity in normal leukocytes and in hematologic malignancies. Blood, 85, 2315-2320.

DE LANGE, T. 2015. A loopy view of telomere evolution. Frontiers in genetics, 6, 321.

DEMISSIE, S., LEVY, D., BENJAMIN, E. J., CUPPLES, L. A., GARDNER, J. P., HERBERT, A., KIMURA, M., LARSON, M. G., MEIGS, J. B. & KEANEY, J. F. 2006. Insulin resistance, oxidative stress, hypertension, and leukocyte telomere length in men from the Framingham Heart Study. Aging cell, 5, 325-330.

EKBLOM, P., LONAI, P. & TALTS, J. F. 2003. Expression and biological role of laminin-1. Matrix Biology, 22, 35-47.

ENARI, M., SAKAHIRA, H., YOKOYAMA, H., OKAWA, K., IWAMATSU, A. AND NAGATA, S., 1998. A caspase-activated DNase that degrades DNA during apoptosis, and its inhibitor ICAD. Nature, 391(6662), pp.43-50.

EPEL, E. S., LIN, J., WILHELM, F. H., WOLKOWITZ, O. M., CAWTHON, R., ADLER, N. E., DOLBIER, C., MENDES, W. B. & BLACKBURN, E. H. 2006. Cell aging in relation to stress arousal and cardiovascular disease risk factors. Psychoneuroendocrinology, 31, 277-287.

FITZPATRICK, A. L., KRONMAL, R. A., GARDNER, J. P., PSATY, B. M., JENNY, N. S., TRACY, R. P., WALSTON, J., KIMURA, M. & AVIV, A. 2007. Leukocyte telomere length and cardiovascular disease in the cardiovascular health study. American journal of epidemiology, 165, 14-21.

GRAHAME, T. J. & SCHLESINGER, R. B. 2012. Oxidative stress-induced telomeric erosion as a mechanism underlying airborne particulate matter-related cardiovascular disease. Particle and fibre toxicology, 9, 21.

GREIDER, C. W. 1998. Telomerase activity, cell proliferation, and cancer. Proceedings of the National Academy of Sciences, 95, 90-92.

HAYFLICK, L. 1965. The limited in vitro lifetime of human diploid cell strains. Experimental cell research, 37, 614-636.

HUZEN, J., PEETERS, W., DE BOER, R.A., MOLL, F.L., WONG, L.S., CODD, V., DE KLEIJN, D.P., DE SMET, B.J., VAN VELDHUISEN, D.J., SAMANI, N.J. AND VAN GILST, W.H., 2011. Circulating leukocyte and carotid atherosclerotic plaque telomere length. Arteriosclerosis, thrombosis, and vascular biology, 31(5), pp. 1219-1225.

ITOH, G., TAMURA, J., SUZUKI, M., SUZUKI, Y., IKEDA, H., KOIKE, M., NOMURA, M., JIE, T. AND ITO, K., 1995. DNA fragmentation of human infarcted myocardial cells demonstrated by the nick end labeling method and DNA agarose gel electrophoresis. The American journal of pathology, 146(6), p.1325.

JOVANOVIC, K., CHETTY, C. J., KHUMALO, T., DA COSTA DIAS, B., FERREIRA, E., MALINDISA, S. T., CAVENEY, R., LETSOLO, B. T. & WEISS, S. F. 2015. Novel patented therapeutic approaches targeting the 37/67 kDa laminin receptor for treatment of cancer and Alzheimer's disease. Expert opinion on therapeutic patents, 25, 567-582.

KVELL K., PONGRÁCZ J., SZÉKELY M., BALASKO M., PÉTERVÁRI E., BAKO G. 2011. Ageing and gene expression, medical scienec, http://www.tankonyvtar.hu/en/tartalom/tamop425/001_1A_Gerontologia_en_book/ch02s04.html, [accessed 17/10/201]

LI, Y., YANG, J. B., JIA, C., YU, G. Y., PEI, Z., LEI, L., WANG, Z. Z., CHANG, C. C., YANG, X. Y. & CHANG, T. Y. 2004. Enhancement of human ACAT1 gene expression to promote the macrophage-derived foam cell formation by dexamethasone. Cell research, 14, 315-323.

MATTHEWS, C., GORENNE, I., SCOTT, S., FIGG, N., KIRKPATRICK, P., RITCHIE, A., GODDARD, M. & BENNETT, M. 2006. Vascular smooth muscle cells undergo telomere-based senescence in human atherosclerosis. Circulation research, 99, 156-164.

MATTHYS, K. & BULT, H. 1997. Nitric oxide function in atherosclerosis. Mediators of inflammation, 6, 3-21. MAZIBUKO Z., OTGAAR T.C., FERREIRA E., & WEISS S.F.T., 2017. Investigating a possible role of LRP::FLAG in a diabetes cell culture model. Unpublished.

NAIDOO, K., MALINDISA, S. T., OTGAAR, T. C., BERNERT, M., DIAS, B. D. C., FERREIRA, E., REUSCH, U., KNACKMUSS, S., LITTLE, M. & WEISS, S. F. 2015. Knock-down of the 37kDa/67kDa laminin receptor LRP/LR impedes telomerase activity. PloS one, 10, e0141618.

NICHOLS, M., TOWNSEND, N., SCARBOROUGH, P. & RAYNER, M. 2012. European cardiovascular disease statistics.

OTGAAR T.C., FERREIRA E., MALINDISA S. BERNERT, M., LETSOLO B. & WEISS S.F.T. 2017. 37kDa LRP::FLAG enhances telomerase activity and reduces senescent markers in vivo, oncotarget, 10.18632/oncotarget.21278.

PIRILLO, A., NORATA, G. D. & CATAPANO, A. L. 2013. LOX-1, OxLDL, and atherosclerosis. Mediators of inflammation, 2013.

SALPEA, K. D. & HUMPHRIES, S. E. 2010. Telomere length in atherosclerosis and diabetes. Atherosclerosis, 209, 35-38.

SAVAGE, D.B., PETERSEN, K.F. AND SHULMAN, G.I., 2007. Disordered lipid metabolism and the pathogenesis of insulin resistance. Physiological reviews, 87(2), pp.507-520.

SHAMMAS, M. A. 2011. Telomeres, lifestyle, cancer, and aging. Current opinion in clinical nutrition and metabolic care, 14, 28.

TEDGUI, A. & MALLAT, Z. 1999. Atherosclerotic plaque formation. La Revue du praticien, 49, 2081-2086.

VAN MEERLOO, J., KASPERS, G. J. & CLOOS, J. 2011. Cell sensitivity assays: the MTT assay. Cancer cell culture: methods and protocols, 237-245.

VANA, K., ZUBER, C., PFLANZ, H., KOLODZIEJCZAK, D., ZEMORA, G., BERGMANN, A.-K. & WEISS, S. 2009. LRP/LR as an alternative promising target in therapy of prion diseases, Alzheimer's disease and cancer. Infectious Disorders-Drug Targets (Formerly Current Drug Targets-Infectious Disorders), 9, 69-80.

VASA, M., BREITSCHOPF, K., ZEIHER, A. M. & DIMMELER, S. 2000. Nitric oxide activates telomerase and delays endothelial cell senescence. Circulation research, 87, 540-542.

WANG, X., SUN, Y., YANG, H., LU, Y. AND LI, L., 2016. Oxidized low-density lipoprotein induces apoptosis in cultured neonatal rat cardiomyocytes by modulating the TLR4/NF-κB pathway. Scientific reports, 6.

WINTERGERST, E.S., JELK, J., RAHNER, C. AND ASMIS, R., 2000. Apoptosis induced by oxidized low density lipoprotein in human monocyte-derived macrophages involves CD36 and activation of caspase-3. The FEBS Journal, 267(19), pp.6050-6059.

YANG, J., ZHANG, L., YU, C., YANG, X.F. AND WANG, H., 2014. Monocyte and macrophage differentiation: circulation inflammatory monocyte as biomarker for inflammatory diseases. Biomarker research, 2(1), p. 1.

YEH, J.-K. & WANG, C.-Y. 2016. Telomeres and Telomerase in Cardiovascular Diseases. Genes, 7, 58. ZUBER, C., LUDEWIGS, H. & WEISS, S. 2007. Therapeutic approaches targeting the prion receptor LRP/LR. Veterinary microbiology, 123, 387-393.

ZETTLER, M.E., PROCIUK, M.A., AUSTRIA, J.A., MASSAELI, H., ZHONG, G. AND PIERCE, G.N., 2003. OxLDL stimulates cell proliferation through a general induction of cell cycle proteins. American Journal of Physiology-Heart and Circulatory Physiology, 284(2), pp.H644-H653.

## EXAMPLE 2 - obesity, insulin resistance and/or diabetes:

### Materials and methods

### Cell culture

The THP-1 human leukemia monocytic cell line was used for Example 2 because when treated with high glucose (HG) it activates monocytes and induces the expression of tumor necrosis factor (TNF)-alpha via oxidant stress and nuclear factor-kB transcription factor (Shanmugum, 2003). This cell line (monocytes) normally grows in suspension but becomes adherent to endothelial cells, which results in endothelial dysfunction seen in type two (II) diabetes individuals, when maintained in high glucose factor (Shanmugum, 2003). The THP-1 cells were cultured in RMPI (Thermofisher Scientific) supplemented with 10% FBS (sigma) to improve growth, 2% penicillin-Streptomycin (Thermofisher Scientific) to prevent bacterial contamination and 1 % MEM non-essential amino acids (lonza). To induce a diabetic state, the THP-1 cells were maintained in 20 mM glucose and were then used in downstream experimental procedures. This generated four different conditions of THP-1 cells: (i). untreated non-transfected, (ii). untreated pCIneo-moLRP::FLAG transfected, (iii). glucose treated non-transfected, and (iv). glucose treated pCIneo-moLRP::FLAG transfected cells.

These cells were cultured in 5% $CO_2$ at 37°C to mimic *in vivo* conditions. Cells were cultured by changing the culturing media twice a week to replenish nutrients. THP-1 cells were also sub-cultured when required (reached confluency of 70-80%) and seeded at appropriate density for downstream experimental procedures. Seeding and sub-culturing of cells involved scrapping of cells and centrifugation at 4500 rpm for 10 minutes. The media was then discarded followed by the re-suspension of the cells with 2 ml of phosphate buffered saline (PBS) to remove excess media. This was then centrifuged at 4500 rpm for 10 minutes before discarding of the supernatant and re-suspension of the cell pellet in fresh cell culture medium. Thereafter, cells were re-suspended with fresh media and transferred back to the flask.

### Transfection

Cells were transfected with pCIneo -moLRP-FLAG plasmid to induce the overexpression of LRP::FLAG protein. This procedure showed that the overexpression of LRP::FLAG increases telomerase activity and reduces lipid content in cells and therefore reduces the diabetic state. The transfection procedure was then performed using Xfect transfection reagent (Clontech). The Xfect reaction buffer was added to 5 μg of pCIneo-moLRP::FLAG plasmid to make a total volume of 100 μl. This was then followed by the addition of 1.5 μl of Xfect polymer which was incubated at room temperature for 10 minutes. This was done to allow the complexes of nanoparticles to form. Thereafter, this Xfect transfection reagent was gently added to cells in the flask. Cells were then incubated for 72 hours at 37°C with a master mix comprising of pCIneo-moLRP-FLAG construct and Xfect reagent. This allowed the transcription and translation of LRP::FLAG thereby resulting in the overexpression of LRP::FLAG protein in the THP-1 cells. In order for the cells to continually transcribe the LRP::FLAG plasmid, cells were occasionally treated with 50 μg/ml geneticin. The transfected cells were then used for downstream experimental procedures.

### Cell counting

Cell counting was done in order to ensure that the same number of cells were used for downstream experiments. The procedure involved staining the cells with filtered Trypan blue (0.4%). This allowed the staining of dead cells blue while viable cells remain unstained as their membrane is still intact thus are selective in the compounds that pass through the membrane. About 10 $\mu$l of the sample was used and mixed with 10 $\mu$l Trypan blue. Thereafter, 10 $\mu$l of this solution was placed on the haemocytometer and the average of viable and dead cells in the 16 small squares was counted under a microscope. To calculate the total number of viable cells the following formula was used:

$$Cell\ concentration\ \left(\frac{cells}{ml}\right) = cells\ per\ 16\ squares\ x\ 10^4\ x\ dilution\ factor$$

The cell concentration was then used to seed an equal concentration of cells into each well of the plates used in order to ensure that the data obtained is accurate.

**Preparation of protein lysate**

Cells were scraped to facilitate cell detachment. The cells were then centrifuged at 6600 rpm for 15 minutes. The media was discarded and the pellet was re-suspended with PBS and centrifuged. The supernatant was then discarded while the pellet was re-suspended in 210 $\mu$l of non-denturating (CHAPS) lysis buffer and incubated at 4°C for 30 minutes. This was then followed by centrifugation in 4°C at 12000 rpm for 20 minutes. The supernatant containing the protein extract was then transferred into a new Eppendorf tube and the pellet containing the cell debris was discarded. The protein lysate was used in downstream applications including telomerase activity and glucose assay. For telomerase activity extracted protein lysate was quantified by Nanodrop spectroscopy, by the Nanodrop ND-1000, to ensure samples were diluted to equal concentrations of 500 ng/$\mu$l of protein.

Bicinchoninic acid™ (BCA) protein assay:

BCA was performed in order to ensure that equal concentrations of protein were used for downstream experiments such as SDS-PAGE and western blotting. The reason why this procedure was used over other protein assays is because the peptide backbone in BCA assay also contributes to colour formation, helping to minimize variability caused by protein compositional differences. It is also very sensitive and it is not affected by different compositions of protein.

The procedure involves resuspension of cell pellets obtained from confluent flasks (70-80% confluency) with 100 $\mu$l of RIPA buffer. RIPA buffer was used to lyse the cells so that the cell lysate would be harvested as the RIPA buffer contains SDS which linearize proteins and gives then an overall negative charge. Thereafter 5 $\mu$l of extracted lysate was diluted with 20 $\mu$l of distilled water and loaded in triplicate in a 96 well plate. BCA standards of 0, 0.2, 0.4, 0.6, 0.8 and 1 mg/ml were prepared and 25 $\mu$l of each standard was loaded in duplicate on the plate. This was then followed by the addition of 200 $\mu$l of BCA into each well which was then incubated for 30 min at 37°C. The plate was then read at 562 nm on an ELISA plate reader. The standards curve was then plotted and used to extrapolate the protein concentration of the samples. The samples were then diluted to a final concentration of 2 mg/ml which was then used for downstream experiments such as SDS PAGE and western blotting.

Sodium Dodecyl Sulphate polyacrylamide gel electrophoresis (SDS- PAGE) and Western Blotting

SDS-PAGE and western blotting were used to analyze the presence and content of protein in the transfected and non-transfected THP-1 cells.

This experimental procedure was used to confirm the overexpression of LRP::FLAG. This was done between the transfected untreated, non-transfected untreated, transfected treated and non-transfected treated THP-1 cells. It was achieved by employing SDS-PAGE to separate the proteins based on size and western blotting which makes use of a primary and secondary antibody for specific protein detection.

**SDS-PAGE**

The extracted lysate was diluted with RIPA buffer to 2mg/ml. RIPA contains SDS which linearizes proteins by adding a negative charge proportional to its size and shape. Since the extracted samples were given an overall negative charge, when exposed to electric current it is separated into bands based on size through the migration of proteins from the anode to the cathode. Since the molecular weight of LRP::FLAG and $\beta$- actin is $\pm$40 kDa and 42 kDa respectively, the sample was loaded on 10% (w/v) polyacrylamide gel.

SDS-PAGE is composed of a separating and stacking gel. The separating gel was made by adding 2.5 ml of 40 % acrylamide with 4.8 ml distilled water, 2.5 ml separating buffer, 100 $\mu$l of 10% APS and 5 $\mu$l TEMED. The separating

gel was then poured between the gel casting plates and covered with isopropanol to remove bubbles as they prevent polymerization. This was then allowed to polymerize. Thereafter, the stacking gel was made by mixing 625 $\mu$l of 40% acrylamide with 3.65 ml of distilled water, 625 $\mu$l stacking buffer, 50 $\mu$l APS and 5 $\mu$l TEMED. Isopropanol was then discarded and the stacking gel was poured onto the polymerised separating gel. The comb was then inserted and the gel was allowed to polymerize. Once the gel had polymerized the plates were placed into the electrophoresis apparatus. The running buffer was then added and the combs were removed. Thereafter 2 mg/ml extracted lysate obtained in section 3.4 was mixed with 1x loading dye and then heated at 95°C for 5 minutes. The heated sample was the vortexed and 5 $\mu$l of the sample was loaded in the wells of the gel along with 2 $\mu$l of the molecular weight marker. The gel was resolved at 120 V for 45 min (until dye front was 1 cm from the bottom of the gel).

## Western Blotting

Electrophoretic transfer was applied on SDS-PAGE gel in order to transfer the resolved proteins from the gel to PVDF membrane. Initially the PVDF membrane was soaked and activated in methanol for 2 min. Thereafter, PVDF membranes were assembled onto the electro-blotting device and the gels were placed on top of the membranes and electro-blotting was conducted for 45 min at 120 V. This was then followed by blocking of the PVDF membrane using 10 ml 3% BSA blocking solution for 1 hour to prevent non-specific binding of the primary antibody. Thereafter, the PVDF membrane was incubated with 2 $\mu$l of primary antibody specific to our protein of interest overnight at 4°C. Unbound primary antibody was washed off with 5 washes of 10 ml PBS Tween for 5 minutes each. This was then followed by the addition of 2 $\mu$l anti-human secondary antibody conjugated to HRP enzyme in 10 ml of 3% BSA for 1 hour at room temperature. Following incubation, 5 washes of 10 ml PBS Tween for 5 minutes each were done to remove unbound secondary antibody. This was followed by the addition of the chemi-luminescent substrate and its signal was detected and analysed using Biorad Chemi-doc software. Densitometry was performed to quantity protein levels and this was done using ImageJ™ software of non-transfected cells and pCIneo-moLRP::FLAG transfected cells.

## Assessing telomerase activity using qPCR in cells overexpressing LRP::FLAG

### Quantitative / real-time PCR

The telomerase activity in transfected untreated, non-transfected untreated, transfected treated and non-transfected treated THP-1 cells was detected to determine if the overexpression of LRP::FLAG enhances telomerase activity in a diabetic environment. The telomerase activity was determined using the telomerase activity detection kit: TRAPeze RT® (Merck Millipore™) according to the manufacturer's protocol. Half reactions were used and telomerase activity was analysed using real time qPCR.

The telomerase activity detection kit is composed of a one buffer, Amplifluor primers and two enzyme system which enables fluorescence detection by real time qPCR. In order for the extracted telomerase to add telomeric repeats to the 3' end of substrate, 2 $\mu$l extracted lysate (contains telomerase) obtained from section 3.2 was incubated with 12.5 $\mu$l of reaction mix at 37°C in a 96 well plate. The reaction mix was also added to standards and positive control. Heat inactivated telomerase was used as the control and water and chaps were used as the negative control. About 2 $\mu$l of these controls were added on a 96 well plate and were incubated with 12.5 $\mu$l control mix at 37°C. The heat inactivated control was done by incubating 30 $\mu$l of extracted lysate at 100 °C for 20 minutes. The reaction mix was done by adding 2.5 $\mu$l 5X TRAPeze RT reaction mix, 0.2 $\mu$l Taq polymerase and 8.8 $\mu$l of PCR grade water into each well. Meanwhile, the control mix was done by adding 2.5 $\mu$l 5X TRAPeze RT control reaction mix, 0.2 $\mu$l Taq polymerase and 7.8 $\mu$l of PCR grade water into each well. The telomerase activity of THP-1 cells was done in duplicate (3 biological repeats and 2 technical repeats) and analysed using the Roche LightCycler 480 which was run for 45 cycles. The generated data was normalised to the standard curve generated from TSR8 samples that was serially diluted. The data was analysed using student t-test.

## Assessing lipid content using red oil staining in cells overexpressing LRP::FLAG.

### Oil Red staining

The lipid content in THP-1 cells was detected to determine if the overexpression of LRP::FLAG reduced the lipid content (diabetic state) in transfected cells mimicking type II diabetes. 8000 cells were seeded per well in a 96 well plate as described in section 3.1. A volume of 100 $\mu$l was used per well for all oil red stain components used in this procedure. The medium was gently removed and the cells were washed once with PBS. Thereafter, formalin was added to the cells and incubated for 45 minutes at room temperature to fix the cells onto the plate. During the incubation time, the oil red working solution was prepared by mixing 3.6 ml of the stock solution with 20 ml isopropanol and 2.4 ml distilled water. Once the incubation time was over, formalin was discarded and the cells were then washed twice using water. Cells were then permeabilized by incubating them with 60% isopropanol for 5 minutes. The 60% isopropanol was then discarded and the cells were then stained with Oil Red Working Solution. This was incubated with gentle agitation for 15 minutes. Thereafter, the oil red o solution was discarded and the cells were washed 3 times with water followed by the addition of hematoxylin to stain the nuclei blue and incubated for 1 minute to enable visualization. The hematoxylin was then discarded and the cells were washed with water 3 times. Cells were then covered with water and viewed under the microscope to visualize and examine the intracellular lipid accumulation. The stain was then quantified by adding 100 $\mu$l of 100% isopropanol and using an ELISA plate reader to accurately read the plate at the wavelength of 572nm. The data was analysed using student t-test.

## Assessing glucose uptake in cells overexpressing LRP::FLAG.

### Colorimetric ELISA

This plate based assay was performed to determine if the overexpression of LRP::FLAG results in an improved glucose uptake in cells cultured in high glucose. We therefore, assessed the glucose uptake using colorimetric ELISA between the untreated pCIneo-moLRP::FLAG transfected, untreated non-transfected, glucose treated non-transfected and glucose treated pCIneo-moLRP::FLAG transfected THP-1 cells. The colorimetric ELISA was chosen as it is quick, highly sensitive and specific. The glucose uptake by THP-1 cells was assessed using the glucose assay kit from Calbiochem®. This kit is composed of buffer, glucose probe, glucose standard and glucose enzyme mix which oxidizes the glucose present and produces a product that reacts with probe to produce colour change. The intensity of the colour is proportional to the amount glucose present in sample.

About 20 $\mu$l of the extracted lysate was then loaded in quadruplicate and 30 $\mu$l of glucose assay buffer was then added to make a total volume of 50 $\mu$l while chaps and glucose buffer were used as the negative control. Thereafter, 50 $\mu$l of glucose reagent mix, which was prepared by mixing 46$\mu$l of glucose assay buffer with 2$\mu$l of glucose probe and 2$\mu$l of glucose enzyme mix, was added to all the plates containing extracted lysate and the controls. This was then incubated at 37 °C for 30 minutes and covered with foil as the reagent is light sensitive. The plate was then read using the enzyme-linked immunosorbent assay (ELISA) plate reader at the absorbance of 570 nm. The data obtained from the ELISA plate reader analysed using the student t-test.

## RESULTS

### LRP::FLAG overexpression in THP-1 cells using western blotting

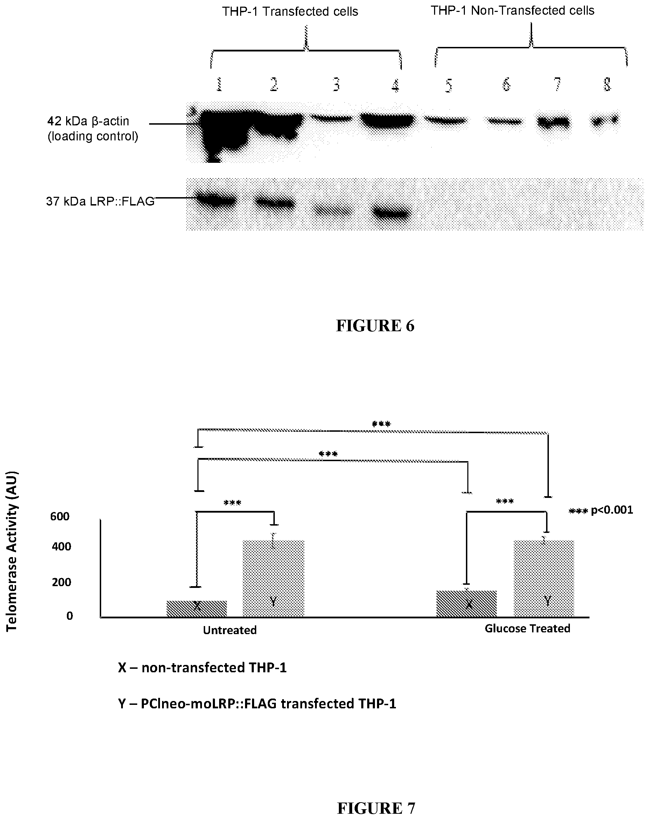

Cell culture model THP- cells were stably transfected with pCIneo-moLRP::FLAG plasmid. This was done to induce the overexpression of LRP::FLAG, which was confirmed using western blot. β-actin was determined as the loading control to confirm that protein loading is the same across the gel and ensure that the results aren't due to loading or protein transfer errors. The β-actin was detected in pCIneo-moLRP::FLAG transfected and non-transfected cells as shown in figure 6. LRP::FLAG was only detected in pCIneo-moLRP::FLAG transfected cells which showed that the transfection procedure was successful (lane 1- 4). Figure 6 shows LRP::FLAG overexpression in THP-1 cells using western blotting. Western blot analyses of β-actin and LRP::FLAG overexpression in THP-1 transfected (lane 1-4) and THP-1 non-transfected cells (lane5-8). The detection of LRP::FLAG protein was achieved using murine anti-FLAG primary antibody and anti-murine IgG secondary antibody coupled to HRP. In addition the loading control (β-actin) was also determined in both the pCIneo-moLRP::FLAG transfected and non-transfected cells. β-actin was detected using monoclonal anti β-actin peroxidase conjugated antibody. LRP::FLAG was determined to be expressed only in THP-1 pCIneo-moLRP::FLAG transfected cells.

### The overexpression of LRP::FLAG significantly increased the telomerase activity in THP-1 cells irrespective of glucose treatment.

The telomeres activity was determined between the untreated pCIneo-moLRP::FLAG transfected, untreated non-transfected, glucose treated pCIneo-moLRP::FLAG transfected and glucose treated non-transfected THP-1 cells. It was found that overexpression of LRP::FLAG resulted in an increase in telomerase activity of untreated THP-1 pCIneo-moLRP::FLAG transfected, glucose treated pCIneo-moLRP::FLAG and glucose treated non-transfected THP-1 cells relative to the untreated non-transfected THP-1 cells (Figure 7). An increase in telomerase activity between glucose treated pCIneo-moLRP::FLAG transfected cells and glucose treated non-transfected cells. Figure 7 shows overexpression of LRP::FLAG significantly increased telomerase activity in THP-1 cells mimicking the diabetic state. Results demonstrated a 4.7 and 1.7 fold increase in telomerase activity of untreated pCIneo-moLRP::FLAG transfected (p= $1 \times 10^{-4}$)

and glucose treated non-transfected THP (p= 8 $\times$ 10$^{-4}$), respectively when compared to untreated non-transfected cells. The Applicant also observed an 4.7 fold significant increase between the glucose treated pCIneo-moLRP::FLAG transfected and untreated non-transfected cells (p= 1 $\times$ 10$^{-4}$) and a 2.8 fold increase between glucose treated non-transfected cells and glucose treated pCIneo-moLRP::FLAG transfected cells (p=4 $\times$ 10$^{-5}$). The data plotted was relative to untreated non-transfected THP-1 cells which were set to 100%.

**Overexpression of LRP::FLAG significantly decreased the lipid content in THP-1 pCIneo-moLRP::FLAG transfected cells.**

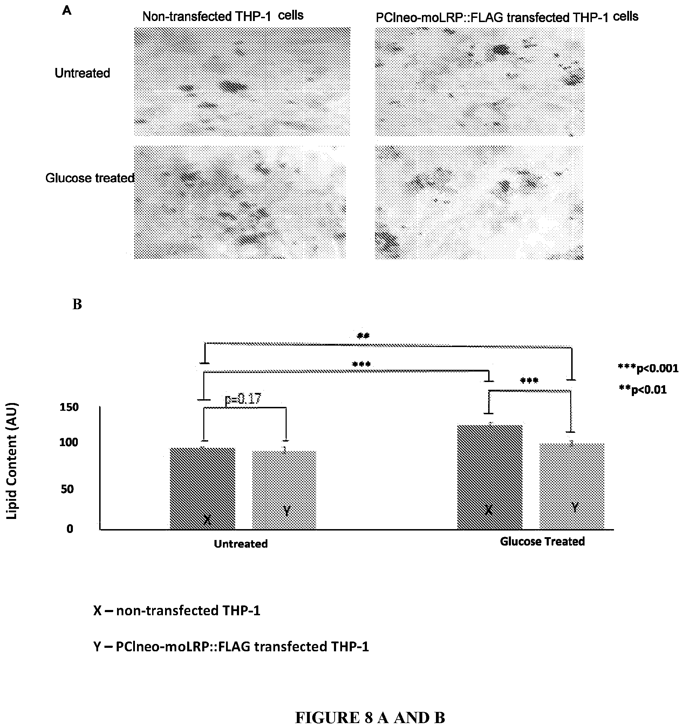

The lipid content was determined in untreated pCIneo-moLRP::FLAG transfected, untreated non-transfected, glucose treated pCIneo-moLRP::FLAG and glucose treated non-transfected THP-1 cells. To assess lipid content, oil red stain was used to stain lipids which was quantified on the ELISA plate reader. The oil red stain demonstrated that overexpression of LRP::FLAG significantly decreased the lipid content shown as a pink stain in glucose treated pCIneo-moLRP::FLAG transfected cells compared to glucose treated non-transfected cells as shown in Figure 8.

Figure 8 shows the overexpression of LRP::FLAG significantly decreased lipid content in THP-1 cells mimicking type 2 diabetic state. (a) Images obtained at 10X magnification illustrate a high lipid content (shown in pink staining) in glucose treated non-transfected cells compared to glucose treated pCIneo-moLRP::FLAG transfected cells, untreated non-transfected cells and untreated pCIneo-moLRP::FLAG transfected cells. The lipid content shown in pink staining of cells of glucose treated pCIneo-moLRP::FLAG transfected THP-1 cells decreased to approximately the same level as the untreated non-transfected THP-1 cells. (b) Glucose treated pCIneo-moLRP::FLAG transfected cells were found to have a significant 1,21 fold decrease in lipid content when compared to glucose treated non-transfected cells (p= 4 $\times$ 10$^{-5}$). We also observed an 1.1 fold decrease between the untreated pCIneo-moLRP::FLAG transfected and untreated non-transfected cells and 1.04 increase in lipid content between untreated non-transfected cells and glucose treated pCIneo-moLRP::FLAG transfected cells (p=0.008). A significant 1.3fold increase in lipid content between untreated non-transfected cells and glucose treated non-transfected cells (p= 3.7 $\times$ 10$^{-6}$). The data plotted was relative to untreated non-transfected THP-1 cells which were set to 100%.

LRP::FLAG overexpression induced an increase in glucose uptake in THP-1 cells mimicking the diabetic state

Overexpression of LRP::FLAG was found to have an effect on the cell's ability to uptake glucose and confirmed by an ELISA based glucose assay. The glucose uptake of the THP-1 cells was determined between the untreated pCIneo-moLRP::FLAG transfected, untreated non-transfected, glucose treated pCIneo-moLRP::FLAG and glucose treated non-transfected cells. The ELISA based glucose assay demonstrated a significant increase glucose uptake in glucose treated pCIneo-moLRP::FLAG transfected cells relative to glucose treated non-transfected cells and between untreated pCIneo-moLRP::FLAG and untreated non-transfected cells. Additionally we observed an increase glucose in media of which the THP-1 untreated non-transfected cells were cultured in relative to the media of THP-1 untreated pCIneo-moLRP::FLAG transfected.

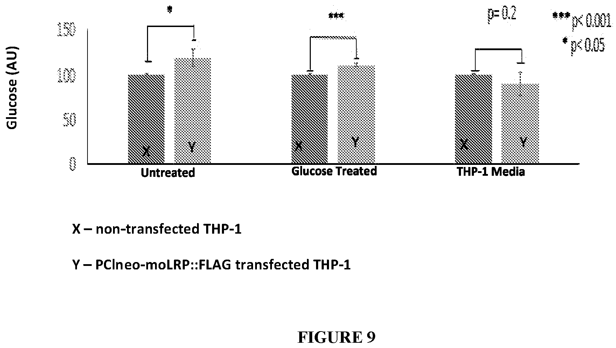

Figure 9 shows LRP::FLAG overexpression induced an increase in glucose uptake in THP-1 cells mimicking the diabetic state. Untreated pCIneo-moLRP::FLAG transfected was determined to have a 1.18 fold increase in glucose uptake relative to untreated non-transfected cells (p= 0.03). Results also demonstrated a 1.1 fold increase between glucose treated pCIneo-moLRP::FLAG transfected cells and glucose treated non-transfected cells (p= 7$\times$ 10$^{-4}$) and a 1.12 fold decrease in glucose content between media of untreated pCIneo-moLRP::FLAG transfected cells and untreated non-transfected cells.

**DISCUSSION & CONCLUSION:**

Western blot revealed LRP::FLAG overexpression in pCIneo-moLRP::FLAG THP-1 transfected cells. This shows that the transfection procedure was successful. The results show an increase in telomerase activity in untreated pCIneo-moLRP::FLAG transfected, glucose treated non-transfected and glucose treated pCIneo-moLRP::FLAG transfected THP-1. This shows that telomerase activity was enhanced, thereby elongating the telomeres. Concomitant with this increase in telomerase activity it was further found that overexpressing LRP::FLAG reduced lipid content in THP-1 cells.

It is further shown that LRP::FLAG-dependent telomerase activation results in a significant reduction in lipid content of glucose treated pCIneo-moLRP::FLAG to approximately the same level as the control (untreated non-transfected and untreated pCIneo-moLRP::FLAG transfect THP-1 cells). Lipid content of the cells in diabetes has been found to be increased which is also associated with insulin resistance (Gavin et al, 2017). Studies have shown that an increase in lipid content leads to insulin resistance (Palmer et al., 2015).

Therefore by overexpressing LRP::FLAG in THP-1 cells telomerase activity was increased. Without being limited to theory, this might have resulted in the reduction of ROS species which also play a role in regulation of lipid metabolism by increasing *sterol regulatory element binding protein* (SREBP) cleavage activating protein expression which activates fatty acid synthase (FAS) (Ryu et al., 2015). FAS is a lipogenic gene which stimulates lipogenesis and this contributes to insulin resistance. We know that increased FFA levels decrease the mitochondrial β-oxidation in skeletal muscle cells and liver cells. This results in increased (DAG) and (LCCoA) which induces serine/threonine phosphorylation of IRS-1 sites, thereby inhibiting IRS-1 phosphorylation and activation of phosphatidylinositol 3-phosphate (PI3P) signaling. (Savage et al. 2007). The inhibition of the activation of PI3P in skeletal results in decreased GLUT4 synthesis and consequently reduced glucose uptake by skeletal muscles. The inhibition of the activation of PI3P in liver cells leads to reduced forkhead box protein O (FOXO) phosphorylation, which in turn results in increased hepatic gluconeogenesis (Savage et al. 2007).Therefore, but without being limited to theory, the overexpression of LRP::FLAG may lead to reduced ROS species which may lead to reduction in lipid metabolism and lipid content as shown. The reduced lipid content possibly might have suppressed insulin resistance possibly by improving insulin sensitivity.

Improved insulin sensitivity and reduced insulin resistance results in increase glucose uptake and reduced glucose production by the cells. Insulin activates the MAPK, PI3K-Akt and CAP/Cbl and small G protein TC10 pathways to increase glucose uptake. The MAPK inhibits gluconeogenesis, PI3K-Akt increases GLUT-4 and CAP/Cbl and small G protein TC10 increases the suppression of lipogenesis (Hajiaghaalipour et al., 2015). This is in line with our results where we observed an increased glucose uptake in THP-1 pCIneo-moLRP-FLAG transfected cells mimicking diabetes type II. This increase glucose uptake by the cells means that the hyperglymemic state (a major hallmark of diabetes) was reduced.

Therefore by using LRP::FLAG, which increases hTERT levels and telomerase activity, it may lead to reduced ROS and thereby results in the reduction of the hyperglycemic state seen in diabetes type II as shown in Figure 7.

Reducing lipid concentration in diabetic cells will improve impaired insulin signaling therein treating and/or preventing obesity, insulin resistance and/or diabetes.

## REFERENCES

Abdul-Ghani, M.A. and DeFronzo, R.A. (2010). Pathogenesis of insulin resistance in skeletal muscle. Biomedical and Biotechnology J. 2010: 1-19

Aburasayn, H., Batran, R.A.I. and Ussher, J.R. (2016). Targeting ceramide metabolism in obesity. Am J Physio Endocrinol Metab. 311(2): E423-435

Blackbum, E. H. (1997) Biochemistry (Moscow, Russ. Fed.). 62: 1196-1201.

Chawla, A., Chawla, R. and Jaggi, S. (2016). Microvascular and macrovascular complications in diabetes mellitus: distinct or continuum? Indian J Endocrinol Metab. 20(4): 546-551.

Chetty, C., Khumalo, T., Da Costa Dias, B., Reusch, U., Knackmuss, S., Little, M. and Wiess, S.F.T. (2014). Anti-LRP/LR Specific Antibody IgG1-iS18 Impedes Adhesion and Invasion of Liver Cancer Cells. PLoS ONE. 9(5): e96268.

Cong, Y., & Shay, J. W. (2008). Actions of human telomerase beyond telomeres. Cell research. 18(7): 725-732.

Cong, Y. S., Wen, J. and S. Bacchetti. (1999). The human telomerase catalytic subunit hTERT: organization of the gene and characterization of the promoter. Hum. Mol. Genet. 8:137-142.

Cong, Y. S., Wright, W.E. and Shay, J.W. (2002). Human Telomerase and Its Regulation. Microbiol Mol Biol Rev. 66(3): 407-425.

Correria-Melo, C. and Passes, T.F (2015). Mitochondria: Are they causal players in cellular senescence? Biochim Biophys Acta. 1847(11):1373-1379.

Counter, C.M., Avilion, A.A., Lefeuvrel, C.E., Stewart, N.G., Greider, C.W., Harley, C.B., and Bacchettil, S. (1992). Telomere shortening associated with chromosome instability is arrested in immortal cells which express telomerase activity. EMBO J. 11: 1921-1929.

Cullinan, K. (2017). Big increase in diabetes death.http://www.news24.com/SouthAfrica/News/tb-diabetes-leading-causes-of-natural-deaths-in-2015-stats-sa-20170228

Cusanelli, E. and Chartrand, P. (2015). Telomeric repeat-containing RNA TERRA: a noncoding RNA connecting telomere biology to genome integrity. Frontiers in Genetics. 6: 143.

Da Costa Dias, B., Jovanovic, K., Gonsalves, D., Moodley, K., Reusch, U., Knackmuss, S., Penny, C., Weinberg, M.S., Little, M., and Weiss, S.F.T. (2013). cytotoxicity. Sci. Rep. 3: 1-8.

Da Costa Dias, B., Jovanovic, K., Gonsalves, D., Moodley, K., Reusch, U., Knackmuss, S., Weinberg, M.S., Little, M., and Weiss, S.F.T. (2014). The 37kDa/67kDa Laminin Receptor acts as a receptor for A b 42 internalization. Sci. Rep. 4: 1-11.

de Jesus, B.B., Schneeberger, K., Vera,E., Tejera, A., Harley, C.B. and Blasco, M.A. (2011). The telomerase activator TA-65 elongates short telomeres and increases health span of adult/old mice without increasing cancer incidence. Ageing cell. 10(4): 604-621

De Lange, T. (2009). How telomeres solve the end protection problem. Science. 329(5955): 948-952.

Dia, J., Carver, M. and Yang, D. (2008). Polymorphism of human telomeric quadruplex structures. Biochimie. 90(8): 1172-1183.

Elks, C.E. and Scott, R.A. (2014). The Long and Short of Telomere Length and Diabetes. Diabetes. 63(1): 65-67.

Esser, N., Legrand-Poels, S., Piette, J., Scheen, A.J. and Paquot, N. (2014). Inflammation as a link between obesity, metabolic syndrome and type 2 diabetes. Diabetes Res

Clin Pract. 105: 141-150

Feng, J., Funk, W.D., Wang, S., Weinrich, S.L., Avilion, A.A., Chiu, C., Adams, R.R., Chang, E., Allsopp, R.C., Yu, J., et al. (1995). The RNA Component of Human Telomerase. Science. 80(269): 1236-1241.

Fidler, I.J. and Radinsky, R. (1990). Genetic control of cancer metastasis. J Natl Cancer Inst. 82: 166-168. Fonseca, V.A. (2009). Defining and Characterizing the progression of type 2 diabetes. Diabetes Care. 23(Suppl 2): S151-S156.

Ford, J.H. (2010). Saturated fatty acid metabolism is key link between cell division, cancer, and senescence in cellular and whole organism aging. Age (Dordr). 32:231-237

Galadari, S., Rahman, A., Pallichankandy, S., Galadari, A. and Thayyullathil, F. (2013). Role of ceramide in diabetes mellitus: evidence and mechanisms. Lipids Health Dis. 12:98

Gauczynski, S., Nikles, D., El-gogo, S., Papy-garcia, D., Alban, S., Barritault, D., Lasme, C.I., and Weiss, S. (2006). The 37-kDa / 67-kDa Laminin Receptor Acts as a Receptor for Infectious Prions and Is Inhibited by Polysulfated Glycanes. JID. 194: 702-709.

Gauczynski, B.Y.S., Hundt, C., Leucht, C., and Weiss, S. (2001a). Interaction of prion proteins with cell surface receptors, olecular chaperones, and other molecules. Adv. Protein Biochem. 57: 229-272.

Gavin, J.R., Weiden, P.J., Xavier Pi-Sunyer, F., and Newcomer, J.W. (2017). New Insight in Diabetes and Psychiatric Illness: Integrating Management Pathophysiology of Insulin Resistance. http// www.medscape.org/viewarticle/442813_2. Retrieved March 2017.

Griffith, J.D., Comeau, L., Rosenfield, S., Stansel, R.M., Bianchi, A., Moss, H., Hill, C. and Carolina, N. (1999). Mammalian Telomeres End in a Large Duplex Loop. Cell. 97: 503-514.

Hajiaghaalipour, F., Khalilpourfarshbafi, M. and Arya, A. (2015). Modulation of Glucose Transporter Protein by Dietary Flavonoids in Type 2 Diabetes Mellitus. Int J Biol Sci. 11(5): 508-524

Harley, C.B., Futcher, A.B., and Greider, C.W. (1990). Telomeres shorten during ageing of human fibroblasts. Nat. 345: 458M60.

Janoušková, E., Nečasová, I., Pavloušková, J., Zimmermann, M., Hluchy, M., Marini, V., Nováková, M. and Hofr, C.(2015). Human Rap1 modulates TRF2 attraction to telomeric DNA. Nucleic Acids Res. 43(5): 2691-2700.

Jovanovic, K., Gonsalves, D., Da, B., Dias, C., Moodley, K., Reusch, U., Knackmuss, S., Penny, C., Weinberg, M.S., Little, M., Wiess, S.F.T. (2013). Anti-LRP / LR specific antibodies and in Alzheimer's disease. Sci Rep. 3:2699.

Jovanovic, K., Loos, B., Da Costa Dias, B., Penny, C., and Weiss, S.F.T. (2014). High Resolution Imaging Study of Interactions between the 37 kDa / 67 kDa Laminin Receptor and APP, Beta- Secretase and Gamma-Secretase in Alzheimer's Disease. PLoS One. 9: 1-10.

Jovanovic, K.,Chetty, C.J., Khumalo, T., Da Costa Dias, B., Ferreira, E., Malindisa, S.T., Caveney, R., Letsolo, B. T. and Weiss, S.F.T. (2015) Novel patented therapeutic approaches targeting the 37kDal67 kDa laminin receptor for treatment of Cancer and Alzheimer's Disease. Expert Opinion on Therapeutic Patents. 25(5):567-582.

Khumalo, T., Ferreira, E., Jovanovic, K., Veale, R. B. and Weiss, S. F. T. (2015). "Knockdown of LRP/LR Induces Apoptosis in Breast and Oesophageal Cancer Cells." PloS one 10: 10: e0139584.

Kirchner, H., Shaheen, F., Kalscheuer, H., Schmid, S. M., Oster, H., & Lehnert, H. (2017). The Telomeric Complex and Metabolic Disease. Genes. 8(7): 176.

Kuhlow, D., Florian, S., von Figura, G., Weimer, S., Schulz, N., Petzke, K. J., Zarse, K., Pfeiffer, A., Rudolph, K. L. and Ristow, M. (2010).Telomerase deficiency impairs glucose metabolism and insulin secretion. Aging 2. Aging (Albany NY). 2(10):650-658

Li, Y. and Tergaonkar, V. (2014). Noncanonical Functions of Telomerase: Implications in Telomerase-Targeted Cancer Therapies. Cancer Res. 74: 1639.

Mbazima, V., Da Costa Dias, B., Omar, A., Jovanovic, K. and Weiss, S.F.T. (2010). Interactions between PrP(c) and other ligands with the 37-kDa/67-kDa laminin receptor. Frontiers in bioscience (Scholar edition). 15:1150-1163.

Mejia, C.F. (2006). Molecular basis of type-2 diabtes. Molecular endocrinology. 87-108.

Mercurio, A.M. (1995). Laminin receptors: Achieving specificity through cooperation. Trends Cell Biol. 5: 419-423.

Meyerson, M., C. M. Counter, E. N. Eaton, L. W. Ellisen, P. Steiner, S. D. Caddle, L. Ziaugra, R. L. Beijersbergen, M. J. Davidoff, Q. Liu, S. Bacchetti, D. A. Haber, and R. A. Weinberg. (1997). hEST2, the putative human telomerase catalytic subunit gene, is upregulated in tumor cells and during immortalization. Cell. 90:785-795.

Miwa, S., Czapiewski, R., Wan, T., Bell, A., Hill, K.N., von Zglinicki, T. and Saretzki, G. (2016). Decreased mTOR signalling reduces mitochondrial ROS in brain via accumulation of the telomerase protein TERT within mitochondria. Aging. 8(10):2551-2567.

Moodley, K., and Weiss, S.F.T. (2013). Downregulation of the non-integrin laminin receptor reduces cellular viability by inducing apoptosis in lung and cervical cancer cells. PLoS One. 8(3): e57409.

Naidoo, K., Malindisa, S.T., Otgaar. T.C., Bernert, M., Da Costa Dias, B., Ferreira, E., Reusch, U., Knackmuss, S., Little, M., Weiss, S.F.T and Letsholo,B.T. (2015) Knock-Down of the 37kDa/67kDa Laminin Receptor LRP/LR Impedes Telomerase Activity. PLoS One 10(11): e0141618.

Naidoo, K., Weiss, S.F.T., and Boitelo, L.T. (2014). University of the Witwatersrand, assignee. Modifying telomerase activity. South African Provisional Patent Application. 2014/08860.

O'Sullivan, R.J. and Karlseder, J. (2010). Telomeres: protecting chromosomes against genome instability. Nat Rev Mol Cell Biol. 11(3): 171-181.

Otgaar, T.C., Ferreira, E., Malindisa, S.T., Bernert, M., Letsolo, B.T., S.F.T. Weiss, S.F.T. (2017). 37 kDa LRP::FLAG enhances telomerase activity and reduces senescent markers in vitro.Oncotarget. 10:18632/0ncotarget.21278.

Palmer, A.K., Tchkonia, T., LeBrasseur, N.K., Chini, E.N., Xu, M. and Kirkland, J.L. (2015). Cellular senescence in type 2 diabetes: A therapeutic opportunity. Diabetes. 65(64):2289-2298.

Ryu, J.M., Lee, H.J., Jung, Y.H., Lee, K.H., Kim, D.I., Kim, J.Y., Ko, S.H., Choi, G.E., Chai, I.I., Song, E.J., Oh, J.Y., Lee, S. and and Han, H.J. (2015). Regulation of Stem Cell Fate by ROS-mediated Alteration of Metabolism. Int J Stem Cells. 8(1): 24-35.

Salpea, K.D. and Humphries. (2010). Telomere length in atherosclerosis and diabetes. Atherosclerosis. 209:35-39.

Santos, J.H., Meyer, J.N. and Van Houten, B. (2006). Mitochondrial localization of telomerase as a determinant for hydrogen peroxide- induced mitochondrial DNA damage and apoptosis. Human Molecular Genetics. 15(11): 1757-1768.

Savage, D.B., Petersen, K.F. and Shulman, G.I. (2007). Disordered lipid metabolism and the pathogenesis of insulin resistance. Physiol Rev. 87: 507-520.

Shay, J.W., Zou, Y., Hiyama, E. and Wright, W.E. (2001). Telomerase and cancer. Human Molecular Genetics. 10(7): 677-685.

Tomas-Loba, A., Flores, I., Ferna, P.J., Cayuela, L., Maraver, A., Serrano, M., Tejera, A., Borra, C., Matheu, A., Klatt, P., et al. (2008). Telomerase Reverse Transcriptase Delays Aging in Cancer-Resistant Mice. Cell. 135: 609-622.

Van Steensel, B., Smogorzewska, A., and De Lange, T. (1998). TRF2 protects human telomeres from end-to-end fusions. Cell. 92: 401-413.

Van Deursen, M. (2014). "The role of senescent cells in ageing". Nature. 509(7501): 439-446.

von Zglinicki, T., Saretzki, G., Docke, W. and Lotze, C. (1995). Mild hyperoxia shortens telomeres and inhibits proliferation of fibroblasts: a model for senescence? Exp Cell Res. 220: 186-193.

von Zglinicki, T. (2002). Oxidative stress shortens telomeres. Trends Biochem. Sci. 27(7): 339-344.

Weinrich, S.L., Pruzan, R., Ma, L., Ouellette, M., Tesmer, V.M., Holt, S.E., Bodnar, A.G., Lichtsteiner, S., Kim, N.W., Trager, J.B., Taylor, R.D., Carlos, R., Andrews, W.H., Wright, W.E., Shay, J.W., Harley, C.B. and Morin, G.B. (1997). Reconstitution of human telomerase with the template RNA component hTR and the catalytic protein subunit hTRT. Nat Genet. 17:498-502.

Weiss, S.F.T.(2017).Bad Boy with a Twist: Targeting the 37kda/67 KDa Laminin Receptor for Treatment of Cancer and Neurodegenerative Diseases and for Changing Telomere Dynamics. Cell Cellular Lif Sci J. 2(2): 000114

Wilcox, G. (2005). Insulin and Insulin resistance. Clin Biochem Rev. 26(2): 19-39.

Xu, Y. (2011). Chemistry in human telomere biology: structure, function and targeting of telomere DNA/RNA. Chem. Soc. Rev. 40: 2719-2740.

Yokoi, T., Fukuo, K., Yasuda, O, et al. (2006). Apoptosis signal-regulating kinase 1 mediates cellular senescence induced by high glucose in endothelial cells. Diabetes 55:1660-1665.

Zhao, J., Zhu, Y., Lin J., Matsuguchi, T., Blackbum, E., Zhang, Y., Cole, S.A., Best, L.G., Lee, E.T. and Howard,B.V. (2013).Short leukocyte telomere length predicts risk of diabetes in American Indians: the Strong Heart Family Study. Diabetes. 63:354-362.

The Applicant believes that the subject matter of the disclosure described herein at least ameliorates one of the disadvantages known in the current state of the art.

**Claims**

1.    A biopharmaceutical agent including a 37 kDa/67 kDa laminin receptor precursor/ high affinity laminin receptor (LRP/LR) and/or a fragment thereof for use in the treatment and/or prevention of obesity, wherein the LRP/LR and/or the fragment thereof being for administration to a target cell of a subject in need thereof, wherein the target cell is at least one of the following group: endothelial cells of blood vessels, smooth muscles cells of blood vessels, pancreatic cells including alpha ($\alpha$) cells, beta ($\beta$) cells, delta ($\delta$), and gamma ($\gamma$) cells, such that in use administration of the biopharmaceutical agent reduces lipid content in the target cell, and wherein the fragment is set forth in SEQ ID NO: 4 or SEQ ID NO: 5.

2. The biopharmaceutical agent for use according to claim 1, wherein the LRP/LR is encapsulated into a delivery means comprising nanoparticles, preferably the nanoparticles being functionalized with chemical, biochemical or biological moieties to ensure site specific delivery to the target cell, wherein the moieties act as ligands to ensure site specific delivery at the target cell

3. The biopharmaceutical agent for use according to claim 2, wherein the delivery means is formulated into a pharmaceutical composition, which pharmaceutical composition including a pharmaceutical carrier for parenteral or non-parenteral administration to the subject.

4. The biopharmaceutical agent for use according to claim 3, wherein the pharmaceutical composition further includes an active pharmaceutical ingredient (API).

5. The biopharmaceutical agent for use according to claim 4, wherein the pharmaceutical composition further includes an anti-oxidant such that in use at the target cell the anti-oxidant scavenges reactive oxygen species.

6. The biopharmaceutical agent for use according to any one of claims 1-5, wherein the LRP/LR comprises a peptide/protein sequence listing as set forth in SEQ ID NO: 1 or SEQ ID NO: 2.

7. The biopharmaceutical agent for use according to any one of claims 1-6, wherein the LRP/LR forms part of a transfecting agent for expressing a 37 kDa/67 kDa laminin receptor precursor/ high affinity laminin receptor (LRP/LR) and/or a fragment thereof, the fragment being set forth in SEQ ID NO: 4 or SEQ ID NO: 5, preferably the transfecting agent is pCIneo-moLRP::FLAG plasmid.

8. The biopharmaceutical agent for use according to any one of claims 2-7, wherein the nanoparticles are polymeric nanoparticles and biodegradable to provide in use a reduced risk of an immunogenic response to said polymeric nanoparticles, and further wherein the polymeric nanoparticles are biocompatible to mitigate risk of any immunogenic response to said polymeric nanoparticles, and further wherein said polymeric nanoparticles are one or more selected from the following group: eudragit, gum arabic, carrageenan, cellulose, hydroxypropyl cellulose (HPC), methylcellulose (MC), hydroxypropylmethylcellulose (HPMC), polylactic-co-glycolic acid (PLGA), chitin, pectin, amylopectic, natural rubber, polyethylene, polypropylene, polystyrene, polyamide, polyacrylonitrile, polyvinyl chloride, polyvinyl alcohol (PVA), polyethylene glycol (PEG), polyethylene oxide (PEO), poly(D-lactide) (PDLA), polylactic acid (PLLA), polygalacturonate, methylcellulose (polyacetals), poly($\varepsilon$-caprolactone), phospholipids, polysaccharides, polyanionic polysaccharides, carboxymethyl cellulose, carboxymethyl amylose, chondroitin-6-sulfate, dermatin sulfate, heparin, heparin sulfate, poly(hydroxyethyl methylacrylate), collagen, fibrinogen, albumin, fibrin, acrylamide, hydroxypropyl methacrylamide-based copolymers, polyacrylamide, poly(N-isopropyl acrylamide) (pNIPAAm), polyvinylpyrrolidone, poly(methacrylic acid-g-ethylene glycol), poly(N-2-hydroxypropyl methacrylamide), poly(glycolic acid) (PGA), poly(lactic acid) (PLA), chitosan, poly(2-hydroxyethylmethacrylate) (HEMA), polyphazene, phosphorylcholine, hyaluronic acid (HA), hydroxyethyl methacrylate (HEMA), methylene-bis-acrylamide (MBAAm), poly(acrylic acid) (PAAc), poly-acrylamide (PAAm), polyacrylonitrile (PAN), polybutylene oxide (PBO), polycaprolactone (PCL), poly(ethylene imine) (PEI), poly(ethyl methacrylate) (PEMA), propylene fumarate (PF), poly(glucosylethyl methacrylate) (PGEMA), poly(hydroxy butyrate) (PHB), poly(hydroxyethyl methacrylate) (PHEMA), poly(hydroxypropyl methacrylamide) (PHPMA), poly(methyl methacrylate) (PMMA), poly(N-vinyl pyrrolidone) (PNVP), polypropylene oxide) (PPO), poly(vinyl acetate) (PVAc), poly(vinyl amine), chondroitin sulfate, dextran sulfate, polylysine, gelatin, carboxymethyl chitin, dextran, agarose, pullulan, polyesters, PEG-PLA-PEG, PEG-PLGA-PEG, PEG-PCL-PEG, PLA-PEG-PLA, poly(PF-co-EG), poly(PEG/PBO-terephthalate), PEG-bis-(PLA-acrylate), PEG6CDs, PEG-g-poly(AAm-co-vinlyamine), poly(NIPAAm-co-AAc), poly(NIPAAm-co-EMA), PNVP, poly(MMA-co-HEMA), poly(AN-co-allyl sulfonate), poly(biscarboxy-phenoxy-phosphazene), poly(GEMA-sulfate), poly(PEG-co-peptides), alginate-g-(PEO-PPO-PEO), poly(PLGA-co-serine), collagen-acrylate, alginate, alginate-acrylate, poly(HPMA-g-peptide), HA-g-NIPAAm, and poly(vinyl methyl ether) (PVME), and/or derivatives of any one or more of the aforementioned.

9. The biopharmaceutical agent for use according to claim 8, wherein the delivery means is formulated for oral delivery and includes at least partially thereabout a coating including an inhibitor of cytochrome P450 3A4 (CYP3A4) selected from the group consisting of polyethylene glycol, polyamine, polymethyl methacrylate and derivatives thereof, and wherein the coating further includes a P-glycoprotein (P-gp) efflux pump inhibitor.

10. A biopharmaceutical agent including a 37 kDa/67 kDa laminin receptor precursor/ high affinity laminin receptor (LRP/LR) and/or a fragment thereof for use in treatment and/or prevention of atherosclerosis, wherein the LRP/LR and/or the fragment thereof being for administration to a target cell of a subject in need thereof, and wherein the target cell is at least one of the following group: endothelial cells of blood vessels and smooth muscles cells of blood vessels, in use the biopharmaceutical agent reduces lipid content in the target cell, and wherein the LRP/LR comprises a peptide/protein sequence listing as set forth in SEQ ID NO: 1 or SEQ ID NO: 2, or a fragment thereof as set forth in SEQ ID NO: 4 or SEQ ID NO: 5.

11. A biopharmaceutical agent including a 37 kDa/67 kDa laminin receptor precursor/ high affinity laminin receptor (LRP/LR) and/or a fragment thereof for use in treatment and/or prevention of insulin resistance and/or diabetes, wherein the LRP/LR and/or the fragment thereof being for administration to a target cell of a subject in need thereof, and wherein the target cell is at least one of the following group: pancreatic cells including alpha ($\alpha$) cells, beta ($\beta$) cells, delta ($\delta$), and gamma ($\gamma$) cells, in use the biopharmaceutical agent reduces lipid content in the target cell, and wherein the LRP/LR comprises a peptide/protein sequence listing as set forth in SEQ ID NO: 1 or SEQ ID NO: 2, or a fragment thereof as set forth in SEQ ID NO: 4 or SEQ ID NO: 5.

## Patentansprüche

1. Biopharmazeutisches Mittel, das einen 37 kDa / 67 kDa Lamininrezeptorvorläufer / Hochaffinitäts-Lamininrezeptor (LRP/LR) und/oder ein Fragment desselben einschließt, zur Verwendung bei der Behandlung und/oder Vorbeugung von Fettleibigkeit, wobei der LRP/LR und/oder das Fragment desselben zur Verabreichung an eine Zielzelle eines Individuums, das dessen bedarf, bestimmt ist, wobei die Zielzelle wenigstens eine aus der folgenden Gruppe ist: Endothelzellen von Blutgefäßen, glatte Muskelzellen von Blutgefäßen, Pankreaszellen einschließlich Alpha- ($\alpha$-)-Zellen, Beta- ($\beta$-)-Zellen, Delta- ($\delta$-), und Gamma- ($\gamma$-)-Zellen, so dass bei Verwendung die Verabreichung des biopharmazeutischen Mittels einen Lipidgehalt in der Zielzelle reduziert, und wobei das Fragment in SEQ ID NO: 4 oder SEQ ID NO: 5 dargelegt ist.

2. Biopharmazeutisches Mittel zur Verwendung nach Patentanspruch 1, wobei das LRP/LR in einem Verabreichungsmittel, welches Nanopartikel umfasst, eingekapselt ist, wobei die Nanopartikel vorzugsweise mit chemischen, biochemischen oder biologischen Gruppen funktionalisiert sind, um eine ortsspezifische Verabreichung an die Zielzelle sicherzustellen, wobei die Gruppen als Liganden fungieren, um die ortsspezifische Verabreichung an die Zielzelle sicherzustellen.

3. Biopharmazeutisches Mittel zur Verwendung nach Patentanspruch 2, wobei das Verabreichungsmittel zu einer pharmazeutischen Zusammensetzung formuliert ist, wobei die pharmazeutische Zusammensetzung einen pharmazeutischen Träger für eine parenterale oder nichtparenterale Verabreichung an das Individuum einschließt.

4. Biopharmazeutisches Mittel zur Verwendung nach Patentanspruch 3, wobei die pharmazeutische Zusammensetzung weiter einen aktiven pharmazeutischen Bestandteil (API) einschließt.

5. Biopharmazeutisches Mittel zur Verwendung nach Patentanspruch 4, wobei die pharmazeutische Zusammensetzung weiter ein Anti-Oxidans einschließt, so dass bei Verwendung an der Zielzelle das Anti-Oxidans reaktive Sauerstoffarten abfängt.

6. Biopharmazeutisches Mittel zur Verwendung nach irgendeinem der Patentansprüche 1-5, wobei das LRP/LR ein Peptid/Proteinsequenzprotokoll wie in SEQ ID NO: 1 oder SEQ ID NO: 2 dargelegt umfasst.

7. Biopharmazeutisches Mittel zur Verwendung nach irgendeinem der Patentansprüche 1-6, wobei das LRP/LR einen Teil eines Transfektionsmittels zur Expression eines 37 kDa / 67 kDa Lamininrezeptorvorläufers / Hochaffinitäts-Lamininrezeptors (LRP/LR) und/oder eines Fragments desselben bildet, wobei das Fragment in SEQ ID NO: 4 oder SEQ ID NO: 5 dargelegt ist, wobei das Transfektionsmittel bevorzugt ein pCIneo-moLRP::FLAG-Plasmid ist.

8. Biopharmazeutisches Mittel zur Verwendung nach irgendeinem der Patentansprüche 2-7, wobei die Nanopartikel polymere Nanopartikel und bioabbaubar sind, um in Verwendung ein reduziertes Risiko einer immunogenen Antwort auf diese polymeren Nanopartikel bereitzustellen, und wobei weiterhin die polymeren Nanopartikel biokompatibel sind, um ein Risiko jeglicher immunogenen Antwort auf diese polymeren Nanopartikel zu verringern, und wobei weiterhin diese polymeren Nanopartikel eines oder mehrere ausgewählt aus der folgenden Gruppe sind: Eudragit, Gummi arabicum, Carrageenan, Cellulose, Hydroxypropylcellulose (HPC), Methylcellulose (MC), Hydroxypropylmethylcellulose (HPMC), Polylactid-co-glycolid (PLGA), Chitin, Pektin, Amylopectin, natürliches Gummi, Polyethylen, Polypropylen, Polystyrol, Polyamid, Polyacrylonitril, Polyvinylchlorid, Polyvinylalkohol (PVA), Polyethylenglycol (PEG), Polyethylenoxid (PEO), Poly(D-Lactid) (PDLA), Polylactid (PLLA), Polygalacturonat, Methylcellulose (Polyacetal), Poly(ε-caprolacton), Phospholipid, Polysaccharid, polyanionisches Polysaccharid, Carboxymethylcellulose, Carboxymethylamylose, Chondroitin-6-sulfat, Dermatinsulfat, Heparin, Heparinsulfat, Poly(hydroxyethylmethylacrylat), Collagen, Fibrinogen, Albumin, Fibrin, Acrylamid, Hydroxypropylmethacrylamid-basierte Copolymere, Polyacrylamid, Poly(N-isopropylacrylamid) (pNIPAAm), Polyvinylpyrrolidon, Poly(methacrylsäure-g-ethylenglycol), Poly(N-2-hydroxypropylmethacrylamid), Poly(glycolsäure) (PGA), Poly(lactid) (PLA), Chitosan, Poly(2-hydroxyethylmethacrylat) (HEMA), Polyphosphazen, Phosphorylcholin, Hyaluronsäure (HA), Hydroxyethylmethacrylat (HEMA), Methylen-bis-Acrylamid (MBAAm), Poly(acrylsäure) (PAAc), Poly-Acrylamid (PAAm), Polyacrylonitril (PAN), Polybutylenoxid (PBO), Polycaprolacton (PCL), Poly(ethylenimin) (PEI), Poly(ethylmethacrylat) (PEMA), Propylenfumarat (PF), Poly(glucosylethylmethacrylat) (PGEMA), Poly(hydroxybutyrat) (PHB), Poly(hydroxyethylmethacrylat) (PHEMA), Poly(hydroxypropylmethacrylamid) (PHPMA), Poly(methylmethacrylat) (PMMA), Poly(N-vinylpyrrolidon) (PNVP), Poly(propylenoxid) (PPO), Poly(vinylacetat) (PVAc), Poly(vinylamin), Chondroitinsulfat, Dextransulfat, Polylysin, Gelatine, Carboxymethylchitin, Dextran, Agarose, Pullulan, Polyester, PEG-PLA-PEG, PEG-PLGA-PEG, PEG-PCL-PEG, PLA-PEG-PLA, Poly(PF-co-EG), Poly(PEG/PBO-Terephthalat), PEG-bis-(PLA-Acrylat), PEG6CDe, PEG-g-Poly(AAm-co-Vinlyamin), Poly(NIPAAm-co-AAc), Poly(NIPAAm-co-EMA), PNVP, Poly(MMA-co-HEMA), Poly(AN-co-allyl-Sulfonat), Poly(biscarboxy-phenoxy-Phosphazen), Poly(GEMA-Sulfat), Poly(PEG-co-Peptide), Alginat-g-(PEO-PPO-PEO), Poly(PLGA-co-Serin), Collagen-Acrylat, Alginat, Alginat-Acrylat, Poly(HPMA-g-Peptid), HA-g-NIPAAm und Poly(vinylmethylether) (PVME), und/oder Derivate irgendeines oder mehrerer der Zuvorgenannten.

**9.** Biopharmazeutisches Mittel zur Verwendung nach Patentanspruch 8, wobei das Verabreichungsmittel für eine orale Verabreichung formuliert ist und zumindest teilweise einen Überzug einschließt, welcher einen Inhibitor von Cytochrom P450 3A4 (CYP3A4) einschließt, der ausgewählt ist aus der Gruppe, bestehend aus Polyethylenglycol, Polyamin, Polymethylmethacrylat und Derivative derselben, und wobei der Überzug weiter einen P-Glycoprotein-(P-gp-)-Effluxpumpen-Inhibitor einschließt.

**10.** Biopharmazeutisches Mittel, das einen 37 kDa / 67 kDa Lamininrezeptorvorläufer / Hochaffinitäts-Lamininrezeptor (LRP/LR) und/oder ein Fragment desselben einschließt, zur Verwendung bei einer Behandlung und/oder Vorbeugung von Atherosklerose, wobei das LRP/LR und/oder das Fragment desselben für eine Verabreichung an eine Zielzelle eines Individuums, das seiner bedarf, bestimmt ist und wobei die Zielzelle wenigstens eine aus der folgenden Gruppe ist: Endothelzellen von Blutgefäßen und glatte Muskelzellen von Blutgefäßen, wobei in Verwendung das biopharmazeutische Mittel einen Lipidgehalt in der Zielzelle reduziert, und wobei das LRP/LR ein Peptid/Proteinsequenzprotokoll umfasst, wie es dargelegt ist in SEQ ID NO: 1 oder SEQ ID NO: 2, oder ein Fragment desselben, wie es ist in SEQ ID NO: 4 oder SEQ ID NO: 5 dargelegt.

**11.** Biopharmazeutisches Mittel, das einen 37 kDa / 67 kDa Lamininrezeptorvorläufer / Hochaffinitäts-Lamininrezeptor (LRP/LR) und/oder ein Fragment desselben einschließt, zur Verwendung bei einer Behandlung und/oder Vorbeugung von Insulinresistenz und/oder Diabetes, wobei das LRP/LR und/oder das Fragment desselben für eine Verabreichung an eine Zielzelle eines Individuums, welche seiner bedarf, bestimmt ist, und wobei die Zielzelle wenigstens eine aus der folgenden Gruppe ist: Pankreaszellen, einschließlich Alpha- (α-)-Zellen, Beta- (β-)-Zellen, Delta- (δ-), und Gamma- (γ-)-Zellen, wobei bei der Verwendung das biopharmazeutische Mittel einen Lipidgehalt in der Zielzelle reduziert, und wobei das LRP/LR ein Peptid/Proteinsequenzprotokoll umfasst, wie es in SEQ ID NO: 1 oder SEQ ID NO: 2 dargelegt ist, oder ein Fragment desselben, wie es in SEQ ID NO: 4 oder SEQ ID NO: 5 dargelegt ist.

**Revendications**

**1.** Agent biopharmaceutique contenant un précurseur de récepteur de laminine / récepteur de laminine de haute affinité (LRP/LR) de 37 kDa / 67 kDa et/ou un fragment de celui-ci pour une utilisation dans le traitement et/ou la prévention de l'obésité, dans lequel le LRP/LR et/ou le fragment de celui-ci est destiné à être administré à une cellule cible d'un sujet en ayant besoin, dans lequel la cellule cible est au moins l'une du groupe suivant : cellules endothéliales de vaisseaux sanguins, cellules musculaires lisses de vaisseaux sanguins, cellules pancréatiques y compris cellules alpha (α), cellules bêta (β), cellules delta (δ) et cellules gamma (γ), de façon que, lors de l'utilisation, l'administration de l'agent biopharmaceutique réduise la teneur en lipides de la cellule cible, et dans lequel le fragment est tel que présenté dans la SEQ ID NO : 4 ou la SEQ ID NO : 5.

**2.** Agent biopharmaceutique pour une utilisation selon la revendication 1, dans lequel le LPR/LR est encapsulé dans un moyen de délivrance comprenant des nanoparticules, les nanoparticules étant de préférence fonctionnalisées avec des fragments chimiques, biochimiques ou biologiques pour assurer une délivrance à la cellule cible avec une spécificité de site, dans lequel les fragments agissent comme des ligands pour assurer une délivrance à la cellule cible avec une spécificité de site.

**3.** Agent biopharmaceutique pour une utilisation selon la revendication 2, dans lequel le moyen de délivrance est formulé en une composition pharmaceutique, laquelle composition pharmaceutique comprend un véhicule pharmaceutique pour une administration au sujet par voie parentérale ou non parentérale.

**4.** Agent biopharmaceutique pour une utilisation selon la revendication 3, dans lequel la composition pharmaceutique contient en outre un ingrédient pharmaceutique actif (API).

**5.** Agent biopharmaceutique pour une utilisation selon la revendication 4, dans lequel la composition pharmaceutique contient en outre un antioxydant de façon que, lors de l'utilisation au niveau de la cellule cible, l'antioxydant piège des espèces oxygénées réactives.

**6.** Agent biopharmaceutique pour une utilisation selon l'une des revendications 1 à 5, dans lequel le LRP/LR comprend une séquence peptidique/protéique telle que présentée dans la SEQ ID NO : 1 ou la SEQ ID NO : 2.

**7.** Agent biopharmaceutique pour une utilisation selon l'une des revendications 1 à 6, dans lequel le LRP/LR forme une partie d'un agent de transfection pour l'expression d'un précurseur de récepteur de laminine / récepteur de laminine de haute affinité (LRP/LR) de 37 kDa / 67 kDa et/ou d'un fragment de celui-ci, le fragment étant présenté dans la SEQ ID NO : 4 ou la SEQ ID NO : 5, de préférence l'agent de transfection est un plasmide pCIneo-mo-LRP::FLAG.

**8.** Agent biopharmaceutique pour une utilisation selon l'une des revendications 2 à 7, dans lequel les nanoparticules sont des nanoparticules polymères et sont biodégradables pour assurer, lors de l'utilisation, un risque réduit de réponse immunogénique auxdites nanoparticules polymères, et en outre dans lequel les nanoparticules polymères sont biocompatibles pour limiter le risque d'une quelconque réponse immunogénique auxdites nanoparticules polymères, et en outre dans lequel lesdites nanoparticules polymères sont une ou plusieurs choisies dans le groupe suivant : eudragit, gomme arabique, carraghénane, cellulose, hydroxypropylcellulose (HPC), méthylcellulose (MC), hydroxypropylméthylcellulose (HPMC), poly(acide lactique-co-glycolique) (PLGA), chitine, pectine, amylopectine, caoutchouc naturel, polyéthylène, polypropylène, polystyrène, polyamide, polyacrylonitrile, poly(chlorure de vinyle), poly(alcool vinylique) (PVA), polyéthylèneglycol (PEG), poly(oxyde d'éthylène) (PEO), poly(D-lactide) (PDLA), poly(acide lactique) (PLLA), polygalacturonate, méthylcellulose (polyacétals), poly(ε-caprolactone), phospholipides, polysaccharides, polysaccharides polyanioniques, carboxyméthylcellulose, carboxyméthylamylose, 6-sulfate de chondroïtine, sulfate de dermatine, héparine, sulfate d'héparine, poly(méthacrylate d'hydroxyéthyle), collagène, fibrinogène, albumine, fibrine, acrylamide, copolymères à base d'hydroxypropylméthacrylamide, polyacrylamide, poly(N-isopropylacrylamide) (pNIPAAm), polyvinylpyrrolidone, poly(acide méthacrylique-g-éthylèneglycol), poly(N-2-hydroxypropylméthacrylamide), poly(acide glycolique) (PGA), poly(acide lactique) (PLA), chitosane, poly(méthacrylate de 2-hydroxyéthyle) (HEMA), polyphazène, phosphorylcholine, acide hyaluronique (HA), méthacrylate d'hydroxyéthyle (HEMA), méthylène-bis-acrylamide (MBAAm), poly(acide acrylique) (PAAc), polyacrylamide (PAAm), polyacrylonitrile (PAN), poly(oxyde de butylène) (PBO), polycaprolactone (PCL), poly(éthylène-imine) (PEI), poly(méthacrylate d'éthyle) (PEMA), fumarate de propylène (PF), poly(méthacrylate de glucosyléthyle) (PGEMA), poly(hydroxybutyrate) (PHB), poly(méthacrylate d'hydroxyéthyle) (PHEMA), poly(hydroxypropylméthacrylamide) (PHPMA), poly(méthacrylate de méthyle) (PMMA), poly(N-vinylpyrrolidone) (PNVP), poly(oxyde de propylène) (PPO), poly(acétate de vinyle) (PVAc), poly(vinylamine), sulfate de chondroïtine, sulfate de dextrane, polylysine, gélatine, carboxyméthyl-chitine, dextrane, agarose, pullulane, polyesters, PEG-PLA-PEG, PEG-PLGA-PEG, PEG-PCL-PEG, PLA-PEG-PLA, poly(PF-co-EG), poly(PEG/PBO-téréphtalate), PEG-bis-(PLA-acrylate), PEG6CDs, PEG-g-poly(AAm-co-vinylamine), poly(NIPAAm-co-AAc), poly(NIPAAm-co-EMA), PNVP, poly(MMA-co-HEMA), poly(AN-co-sulfonate d'allyle), poly(biscarboxy-phénoxy-phosphazène), poly(GEMA-sulfate), poly(PEG-co-peptides), alginate-g-(PEO-PPO-PEO), poly(PLGA-co-sérine), collagène-acrylate, alginate, alginate-acrylate, poly(HPMA-g-peptide), HA-g-NIPAAm, et poly(vinylméthyléther) (PVME), et/ou les dérivés de l'un quelconque ou plusieurs des précédents.

9. Agent biopharmaceutique pour une utilisation selon la revendication 8, dans lequel le moyen de délivrance est formulé pour une délivrance par voie orale et contient, au moins partiellement autour de celui-ci un enrobage contenant un inhibiteur de cytochrome P450 3A4 (CYP3A4) choisi dans le groupe constitué par un polyéthylèneglycol, une polyamine, un poly(méthacrylate de méthyle) et leurs dérivés, et dans lequel l'enrobage contient en outre un inhibiteur de pompe d'efflux P-glycoprotéine (P-gp).

10. Agent biopharmaceutique contenant un précurseur de récepteur de laminine / récepteur de laminine de haute affinité (LRP/LR) de 37 kDa / 67 kDa et/ou un fragment de celui-ci pour une utilisation dans le traitement et/ou la prévention de l'athérosclérose, dans lequel le LRP/LR et/ou le fragment de celui-ci est destiné à être administré à une cellule cible d'un sujet en ayant besoin, et dans lequel la cellule cible est au moins l'une du groupe suivant : cellules endothéliales de vaisseaux sanguins et cellules musculaires lisses de vaisseaux sanguins, lors de l'utilisation l'agent biopharmaceutique réduit la teneur en lipides de la cellule cible, et dans lequel le LRP/LR comprend une séquence peptidique/protéique listée telle que présentée dans la SEQ ID NO : 1 ou la SEQ ID NO : 2, ou un fragment de celle-ci telle que présentée dans la SEQ ID NO : 4 ou la SEQ ID NO : 5.

11. Agent biopharmaceutique contenant un précurseur de récepteur de laminine / récepteur de laminine de haute affinité (LRP/LR) de 37 kDa / 67 kDa et/ou un fragment de celui-ci pour une utilisation dans le traitement et/ou la prévention de la résistance à l'insuline et/ou du diabète, dans lequel le LRP/LR et/ou le fragment de celui-ci est destiné à être administré à une cellule cible d'un sujet en ayant besoin, et la cellule cible est au moins l'une du groupe suivant : cellules pancréatiques y compris cellules alpha ($\alpha$), cellules bêta ($\beta$), cellules delta ($\delta$) et cellules gamma ($\gamma$), lors de l'utilisation l'agent biopharmaceutique réduit la teneur en lipides de la cellule cible, et dans lequel le LRP/LR comprend une séquence peptidique/protéique listée telle que présentée dans la SEQ ID NO : 1 ou la SEQ ID NO : 2, ou un fragment de celle-ci telle que présentée dans la SEQ ID NO : 4 ou la SEQ ID NO : 5.

**FIGURE 1**

**FIGURE 2**

**FIGURE 3**

Untransfected untreated THP-1 cells

Untransfected treated THP-1 cells

Transfected untreated THP-1 cells

Transfected treated THP-1 cells

**FIGURE 4**

**(A)**

Untransfected untreated THP-

Untransfected treated THP-1

Transfected untreated THP-1

Transfected treated THP-1

**(B)**

**FIGURE 5 A and B**

THP-1 Transfected cells        THP-1 Non-Transfected cells

42 kDa β-actin
(loading control)

37 kDa LRP::FLAG

**FIGURE 6**

X – non-transfected THP-1

Y – PCIneo-moLRP::FLAG transfected THP-1

**FIGURE 7**

A

Non-transfected THP-1 cells         PClneo-moLRP::FLAG transfected THP-1 cells

Untreated

Glucose treated

B

X – non-transfected THP-1

Y – PCIneo-moLRP::FLAG transfected THP-1

**FIGURE 8 A AND B**

X – non-transfected THP-1

Y – PClneo-moLRP::FLAG transfected THP-1

**FIGURE 9**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017077516 A2
- JP 2018027935 A
- JP 2015155383 A
- ZA 201408860, Naidoo, K., Weiss, S.F.T., and Boitelo, L.T.

**Non-patent literature cited in the description**

- **AUBERT, G. ; LANSDORP, P. M.** *Telomeres and aging. Physiological reviews,* 2008, vol. 88, 557-579
- **BENTZON, J. F. ; OTSUKA, F. ; VIRMANI, R. ; FALK, E.** Mechanisms of plaque formation and rupture. *Circulation research,* 2014, vol. 114, 1852-1866
- **BLACKBURN, E. H. ; GREIDER, C. W. ; SZOSTAK, J. W.** Telomeres and telomerase: the path from maize, Tetrahymena and yeast to human cancer and aging. *Nature medicine,* 2006, vol. 12, 1133-1138
- **BLASCO, M.A.** Mice with bad ends: mouse models for the study of telomeres and telomerase in cancer and aging. *The EMBO journal,* 2005, vol. 24 (6), 1095-1103
- **BREITSCHOPF, K. ; ZEIHER, A.M. ; DIMMELER, S.** Pro-atherogenic factors induce telomerase inactivation in endothelial cells through an Akt-dependent mechanism. *FEBS letters,* 2001, vol. 493 (1), 21-25
- **CHEN, Q ; VAZQUEZ, E. J. ; MOGHADDAS, S. ; HOPPEL, C. L. ; LESNEFSKY, E. J.** Production of reactive oxygen species by mitochondria central role of complex III. *Journal of Biological Chemistry,* 2003, vol. 278, 36027-36031
- **CONG, Y.-S. ; WRIGHT, W. E. ; SHAY, J. W.** Human telomerase and its regulation. *Microbiology and molecular biology reviews,* 2002, vol. 66, 407-425
- **CONTE, M.S. ; POMPOSELLI, F.B. ; CLAIR, D.G ; GERAGHTY, P.J. ; MCKINSEY, J.F. ; MILLS, J.L. ; MONETA, G.L. ; MURAD, M.H. ; POWELL, R.J. ; REED, A.B.** Society for Vascular Surgery practice guidelines for atherosclerotic occlusive disease of the lower extremities: management of asymptomatic disease and claudication. *Journal of vascular surgery,* 2015, vol. 61 (3), 2S-41S
- **COUNTER, C. M ; GUPTA, J. ; HARLEY, C. B. ; LEBER, B. ; BACCHETTI, S.** Telomerase activity in normal leukocytes and in hematologic malignancies. *Blood,* 1995, vol. 85, 2315-2320

- **DE LANGE, T.** A loopy view of telomere evolution. *Frontiers in genetics,* 2015, vol. 6, 321
- **DEMISSIE, S. ; LEVY, D ; BENJAMIN, E. J. ; CUPPLES, L. A. ; GARDNER, J. P ; HERBERT, A. ; KIMURA, M. ; LARSON, M. G. ; MEIGS, J. B. ; KEANEY, J. F.** Insulin resistance, oxidative stress, hypertension, and leukocyte telomere length in men from the Framingham Heart Study. *Aging cell,* 2006, vol. 5, 325-330
- **EKBLOM, P. ; LONAI, P. ; TALTS, J. F.** Expression and biological role of laminin-1. *Matrix Biology,* 2003, vol. 22, 35-47
- **ENARI, M. ; SAKAHIRA, H. ; YOKOYAMA, H. ; OKAWA, K. ; IWAMATSU, A. ; NAGATA, S.** A caspase-activated DNase that degrades DNA during apoptosis, and its inhibitor ICAD. *Nature,* 1998, vol. 391 (6662), 43-50
- **EPEL, E. S. ; LIN, J. ; WILHELM, F. H ; WOLKOWITZ, O. M. ; CAWTHON, R. ; ADLER, N. E. ; DOLBIER, C ; MENDES, W. B. ; BLACKBURN, E. H.** Cell aging in relation to stress arousal and cardiovascular disease risk factors. *Psychoneuroendocrinology,* 2006, vol. 31, 277-287
- **WALSTON, J. ; KIMURA, M. ; AVIV, A.** Leukocyte telomere length and cardiovascular disease in the cardiovascular health study. *American journal of epidemiology,* 2007, vol. 165, 14-21
- **GRAHAME, T. J. ; SCHLESINGER, R. B.** *Particle and fibre toxicology,* 2012, vol. 9 (21
- **GREIDER, C. W.** Telomerase activity, cell proliferation, and cancer. *Proceedings of the National Academy of Sciences,* 1998, vol. 95, 90-92
- **HAYFLICK, L.** The limited in vitro lifetime of human diploid cell strains. *Experimental cell research,* 1965, vol. 37, 614-636
- **HUZEN, J. ; PEETERS, W. ; DE BOER, R.A. ; MOLL, F.L. ; WONG, L.S. ; CODD, V. ; DE KLEIJN, D.P. ; DE SMET, B.J ; VAN VELDHUISEN, D.J. ; SAMANI, N.J.** Circulating leukocyte and carotid atherosclerotic plaque telomere length. *Arteriosclerosis, thrombosis, and vascular biology,* 2011, vol. 31 (5), 1219-1225

- **ITOH, G. ; TAMURA, J. ; SUZUKI, M. ; SUZUKI, Y. ; IKEDA, H. ; KOIKE, M. ; NOMURA, M ; JIE, T. ; ITO, K.** DNA fragmentation of human infarcted myocardial cells demonstrated by the nick end labeling method and DNA agarose gel electrophoresis. *The American journal of pathology,* 1995, vol. 146 (6), 1325
- **JOVANOVIC, K. ; CHETTY, C. J. ; KHUMALO, T. ; DA COSTA DIAS, B. ; FERREIRA, E ; MALINDISA, S. T. ; CAVENEY, R. ; LETSOLO, B. T. ; WEISS, S. F.** Novel patented therapeutic approaches targeting the 37/67 kDa laminin receptor for treatment of cancer and Alzheimer's disease. *Expert opinion on therapeutic patents,* 2015, vol. 25, 567-582
- **KVELL K. ; PONGRÁCZ J. ; SZÉKELY M. ; BALASKO M. ; PÉTERVÁRI E. ; BAKO G.** *Ageing and gene expression,* 2011, http://www.tankonyvtar.hu/en/tartalom/tamop425/001_1A_Gerontologia_en_book/ch02s04.html
- **LI, Y. ; YANG, J. B. ; JIA, C. ; YU, G. Y. ; PEI, Z. ; LEI, L. ; WANG, Z. Z. ; CHANG, C. C. ; YANG, X. Y. ; CHANG, T. Y.** Enhancement of human ACAT1 gene expression to promote the macrophage-derived foam cell formation by dexamethasone. *Cell research,* 2004, vol. 14, 315-323
- **MATTHEWS, C ; GORENNE, I. ; SCOTT, S. ; FIGG, N. ; KIRKPATRICK, P. ; RITCHIE, A. ; GODDARD, M. ; BENNETT, M.** Vascular smooth muscle cells undergo telomere-based senescence in human atherosclerosis. *Circulation research,* 2006, vol. 99, 156-164
- **MATTHYS, K. ; BULT, H.** Nitric oxide function in atherosclerosis. *Mediators of inflammation,* 1997, vol. 6, 3-21
- **MAZIBUKO Z. ; OTGAAR T.C. ; FERREIRA E. ; WEISS S.F.T.** *Investigating a possible role of LRP::FLAG in a diabetes cell culture model.,* 2017
- **NAIDOO, K. ; MALINDISA, S. T. ; OTGAAR, T. C. ; BERNERT, M. ; DIAS, B. D. C. ; FERREIRA, E. ; REUSCH, U. ; KNACKMUSS, S ; LITTLE, M. ; WEISS, S. F.** Knock-down of the 37kDa/67kDa laminin receptor LRP/LR impedes telomerase activity. *PloS one,* 2015, vol. 10, e0141618
- **NICHOLS, M. ; TOWNSEND, N. ; SCARBOROUGH, P. ; RAYNER, M.** *European cardiovascular disease statistics.,* 2012
- **OTGAAR T.C. ; FERREIRA E. ; MALINDISA S. ; BERNERT, M. ; LETSOLO B. ; WEISS S.F.T.** 37kDa LRP::FLAG enhances telomerase activity and reduces senescent markers in vivo. *oncotarget,* 2017
- **PIRILLO, A. ; NORATA, G. D. ; CATAPANO, A. L.** LOX-1, OxLDL, and atherosclerosis. *Mediators of inflammation,* 2013
- **SALPEA, K. D. ; HUMPHRIES, S. E.** Telomere length in atherosclerosis and diabetes. *Atherosclerosis,* 2010, vol. 209, 35-38
- **SAVAGE, D.B. ; PETERSEN, K.F. ; SHULMAN, G.I.** Disordered lipid metabolism and the pathogenesis of insulin resistance. *Physiological reviews,* 2007, vol. 87 (2), 507-520
- **SHAMMAS, M. A.** Telomeres, lifestyle, cancer, and aging. *Current opinion in clinical nutrition and metabolic care,* 2011, vol. 14 (28
- **TEDGUI, A. ; MALLAT, Z.** Atherosclerotic plaque formation. *La Revue du praticien,* 1999, vol. 49, 2081-2086
- **VAN MEERLOO, J. ; KASPERS, G. J. ; CLOOS, J.** Cell sensitivity assays: the MTT assay. *Cancer cell culture: methods and protocols,* 2011, 237-245
- **VANA, K. ; ZUBER, C. ; PFLANZ, H. ; KOLODZIEJCZAK, D. ; ZEMORA, G. ; BERGMANN, A.-K. ; WEISS, S.** LRP/LR as an alternative promising target in therapy of prion diseases, Alzheimer's disease and cancer. *Infectious Disorders-Drug Targets (Formerly Current Drug Targets-Infectious Disorders),* 2009, vol. 9, 69-80
- **VASA, M. ; BREITSCHOPF, K. ; ZEIHER, A. M. ; DIMMELER, S.** Nitric oxide activates telomerase and delays endothelial cell senescence. *Circulation research,* 2000, vol. 87, 540-542
- **WANG, X. ; SUN, Y. ; YANG, H. ; LU, Y. ; LI, L.** Oxidized low-density lipoprotein induces apoptosis in cultured neonatal rat cardiomyocytes by modulating the TLR4/NF-κB pathway. *Scientific reports,* 2016, vol. 6
- **WINTERGERST, E.S ; JELK, J. ; RAHNER, C. ; ASMIS, R.** Apoptosis induced by oxidized low density lipoprotein in human monocyte-derived macrophages involves CD36 and activation of caspase-3. *The FEBS Journal,* 2000, vol. 267 (19), 6050-6059
- **YANG, J. ; ZHANG, L. ; YU, C. ; YANG, X.F. ; WANG, H.** Monocyte and macrophage differentiation: circulation inflammatory monocyte as biomarker for inflammatory diseases. *Biomarker research,* 2014, vol. 2 (1), 1
- **YEH, J.-K. ; WANG, C.-Y.** Telomeres and Telomerase in Cardiovascular Diseases. *Genes,* 2016, vol. 7 (58
- **ZUBER, C. ; LUDEWIGS, H. ; WEISS, S.** Therapeutic approaches targeting the prion receptor LRP/LR. *Veterinary microbiology,* 2007, vol. 123, 387-393
- **ZETTLER, M.E. ; PROCIUK, M.A. ; AUSTRIA, J.A. ; MASSAELI, H. ; ZHONG, G. ; PIERCE, G.N.** OxLDL stimulates cell proliferation through a general induction of cell cycle proteins. *American Journal of Physiology-Heart and Circulatory Physiology,* 2003, vol. 284 (2), H644-H653
- **ABDUL-GHANI, M.A. ; DEFRONZO, R.A.** Pathogenesis of insulin resistance in skeletal muscle. *Biomedical and Biotechnology J. 2010,* 2010, 1-19

- **ABURASAYN, H. ; BATRAN, R.A.I. ; USSHER, J.R.** Targeting ceramide metabolism in obesity. *Am J Physio Endocrinol Metab.,* 2016, vol. 311 (2), E423-435
- **BLACKBUM, E. H.** *Biochemistry (Moscow, Russ. Fed.).,* 1997, vol. 62, 1196-1201
- **CHAWLA, A. ; CHAWLA, R. ; JAGGI, S.** Microvascular and macrovascular complications in diabetes mellitus: distinct or continuum?. *Indian J Endocrinol Metab.,* 2016, vol. 20 (4), 546-551
- **CHETTY, C. ; KHUMALO, T ; DA COSTA DIAS, B. ; REUSCH, U. ; KNACKMUSS, S. ; LITTLE, M. ; WIESS, S.F.T.** Anti-LRP/LR Specific Antibody IgG1-iS18 Impedes Adhesion and Invasion of Liver Cancer Cells. *PLoS ONE.,* 2014, vol. 9 (5), e96268
- **CONG, Y. ; SHAY, J. W.** Actions of human telomerase beyond telomeres. *Cell research,* 2008, vol. 18 (7), 725-732
- **CONG, Y. S. ; WEN, J. ; S. BACCHETTI.** The human telomerase catalytic subunit hTERT: organization of the gene and characterization of the promoter. *Hum. Mol. Genet.,* 1999, vol. 8, 137-142
- **CONG, Y. S. ; WRIGHT, W.E. ; SHAY, J.W.** Human Telomerase and Its Regulation. *Microbiol Mol Biol Rev.,* 2002, vol. 66 (3), 407-425
- **CORRERIA-MELO, C. ; PASSES, T.F.** *Biochim Biophys Acta,* 2015, vol. 1847 (11), 1373-1379
- **COUNTER, C.M. ; AVILION, A.A. ; LEFEUVREL, C.E. ; STEWART, N.G ; GREIDER, C.W. ; HARLEY, C.B. ; BACCHETTIL, S.** Telomere shortening associated with chromosome instability is arrested in immortal cells which express telomerase activity. *EMBO J.,* 1992, vol. 11, 1921-1929
- **CULLINAN, K.** *Big increase in diabetes death,* 2017, http://www.news24.com/SouthAfrica/News/tb-diabetes-leading-causes-of-natural-deaths-in-2015-stats-sa-20170228
- **CUSANELLI, E. ; CHARTRAND, P.** Telomeric repeat-containing RNA TERRA: a noncoding RNA connecting telomere biology to genome integrity. *Frontiers in Genetics,* 2015, vol. 6, 143
- **DA COSTA DIAS, B. ; JOVANOVIC, K. ; GONSALVES, D. ; MOODLEY, K. ; REUSCH, U. ; KNACKMUSS, S. ; PENNY, C. ; WEINBERG, M.S. ; LITTLE, M ; WEISS, S.F.T.** cytotoxicity. *Sci. Rep.,* 2013, vol. 3, 1-8
- **DA COSTA DIAS, B. ; JOVANOVIC, K. ; GONSALVES, D. ; MOODLEY, K. ; REUSCH, U. ; KNACKMUSS, S ; WEINBERG, M.S. ; LITTLE, M. ; WEISS, S.F.T.** The 37kDa/67kDa Laminin Receptor acts as a receptor for A b 42 internalization. *Sci. Rep.,* 2014, vol. 4, 1-11
- **DE JESUS, B.B. ; SCHNEEBERGER, K ; VERA,E ; TEJERA, A. ; HARLEY, C.B. ; BLASCO, M.A.** The telomerase activator TA-65 elongates short telomeres and increases health span of adult/old mice without increasing cancer incidence. *Ageing cell.,* 2011, vol. 10 (4), 604-621
- **DE LANGE, T.** How telomeres solve the end protection problem. *Science,* 2009, vol. 329 (5955), 948-952
- **DIA, J. ; CARVER, M. ; YANG, D.** Polymorphism of human telomeric quadruplex structures. *Biochimie.,* 2008, vol. 90 (8), 1172-1183
- **ELKS, C.E. ; SCOTT, R.A.** The Long and Short of Telomere Length and Diabetes. *Diabetes.,* 2014, vol. 63 (1), 65-67
- **ESSER, N. ; LEGRAND-POELS, S ; PIETTE, J. ; SCHEEN, A.J ; PAQUOT, N.** Inflammation as a link between obesity, metabolic syndrome and type 2 diabetes. *Diabetes Res,* 2014
- **FENG, J. ; FUNK, W.D. ; WANG, S. ; WEINRICH, S.L. ; AVILION, A.A. ; CHIU, C. ; ADAMS, R.R. ; CHANG, E. ; ALLSOPP, R.C. ; YU, J. et al.** The RNA Component of Human Telomerase. *Science,* 1995, vol. 80 (269), 1236-1241
- **FIDLER, I.J. ; RADINSKY, R.** Genetic control of cancer metastasis. *J Natl Cancer Inst.,* 1990, vol. 82, 166-168
- **FONSECA, V.A.** Defining and Characterizing the progression of type 2 diabetes. *Diabetes Care.,* 2009, vol. 23 (2), S151-S156
- **FORD, J.H.** Saturated fatty acid metabolism is key link between cell division, cancer, and senescence in cellular and whole organism aging. *Age (Dordr),* 2010, vol. 32, 231-237
- **GALADARI, S. ; RAHMAN, A. ; PALLICHANKANDY, S. ; GALADARI, A. ; THAYYULLATHIL, F.** Role of ceramide in diabetes mellitus: evidence and mechanisms. *Lipids Health Dis.,* 2013, vol. 12 (98
- **GAUCZYNSKI, S. ; NIKLES, D. ; EL-GOGO, S ; PAPY-GARCIA, D. ; ALBAN, S. ; BARRITAULT, D. ; LASME, C.I. ; WEISS, S.** The 37-kDa / 67-kDa Laminin Receptor Acts as a Receptor for Infectious Prions and Is Inhibited by Polysulfated Glycanes. *JID.,* 2006, vol. 194, 702-709
- **GAUCZYNSKI, B.Y.S. ; HUNDT, C. ; LEUCHT, C. ; WEISS, S.** Interaction of prion proteins with cell surface receptors, olecular chaperones, and other molecules. *Adv. Protein Biochem.,* 2001, vol. 57, 229-272
- **GAVIN, J.R. ; WEIDEN, P.J. ; XAVIER PI-SUNYER, F. ; NEWCOMER, J.W.** *New Insight in Diabetes and Psychiatric Illness: Integrating Management Pathophysiology of Insulin Resistance.,* 2017, http//www.medscape.org/viewarticle/442813_2.

- **GRIFFITH, J.D. ; COMEAU, L. ; ROSENFIELD, S. ; STANSEL, R.M. ; BIANCHI, A. ; MOSS, H. ; HILL, C. ; CAROLINA, N.** Mammalian Telomeres End in a Large Duplex Loop. *Cell,* 1999, vol. 97, 503-514
- **HAJIAGHAALIPOUR, F. ; KHALILPOURFARSH-BAFI, M. ; ARYA, A.** Modulation of Glucose Transporter Protein by Dietary Flavonoids in Type 2 Diabetes Mellitus. *Int J Biol Sci.,* 2015, vol. 11 (5), 508-524
- **HARLEY, C.B. ; FUTCHER, A.B. ; GREIDER, C.W.** Telomeres shorten during ageing of human fibroblasts. *Nat.,* 1990, vol. 345, 458M60
- **JANOUŠKOVÁ, E. ; NEČASOVÁ, I. ; PAVLOUŠK-OVÁ, J. ; ZIMMERMANN, M. ; HLUCHY, M. ; MARINI, V. ; NOVÁKOVÁ, M. ; HOFR, C.** Human Rap1 modulates TRF2 attraction to telomeric DNA. *Nucleic Acids Res.,* 2015, vol. 43 (5), 2691-2700
- **JOVANOVIC, K. ; GONSALVES, D ; DA, B. ; DIAS, C. ; MOODLEY, K. ; REUSCH, U. ; KNACKMUSS, S. ; PENNY, C. ; WEINBERG, M.S. ; LITTLE, M.** Anti-LRP / LR specific antibodies and in Alzheimer 's disease. *Sci Rep.,* 2013, vol. 3, 2699
- **JOVANOVIC, K ; LOOS, B. ; DA COSTA DIAS, B ; PENNY, C. ; WEISS, S.F.T.** High Resolution Imaging Study of Interactions between the 37 kDa / 67 kDa Laminin Receptor and APP , Beta- Secretase and Gamma-Secretase in Alzheimer ' s Disease. *PLoS One.,* 2014, vol. 9, 1-10
- **JOVANOVIC, K ; CHETTY, C.J. ; KHUMALO, T. ; DA COSTA DIAS, B. ; FERREIRA, E. ; MALINDISA, S.T. ; CAVENEY, R. ; LETSOLO, B. T. ; WEISS, S.F.T.** Expert Opinion on Therapeutic Patents., 2015, vol. 25 (5), 567-582
- **KHUMALO, T. ; FERREIRA, E. ; JOVANOVIC, K. ; VEALE, R. B. ; WEISS, S. F. T.** Knockdown of LRP/LR Induces Apoptosis in Breast and Oesophageal Cancer Cells. *PloS one,* 2015, vol. 10 (10), e0139584
- **KIRCHNER, H. ; SHAHEEN, F. ; KALSCHEUER, H ; SCHMID, S. M. ; OSTER, H. ; LEHNERT, H.** The Telomeric Complex and Metabolic Disease. *Genes,* 2017, vol. 8 (7), 176
- **KUHLOW, D. ; FLORIAN, S. ; VON FIGURA, G. ; WEIMER, S. ; SCHULZ, N. ; PETZKE, K. J. ; ZARSE, K. ; PFEIFFER, A. ; RUDOLPH, K. L. ; RISTOW, M.** *Aging (Albany NY),* 2010, vol. 2 (10), 650-658
- **LI, Y. ; TERGAONKAR, V.** *Cancer Res.,* 2014, vol. 74, 1639
- **MBAZIMA, V. ; DA COSTA DIAS, B. ; OMAR, A. ; JOVANOVIC, K. ; WEISS, S.F.T.** Interactions between PrP(c) and other ligands with the 37-kDa/67-kDa laminin receptor. *Frontiers in bioscience,* 2010, vol. 15, 1150-1163
- **MEJIA, C.F.** Molecular basis of type-2 diabtes. *Molecular endocrinology.,* 2006, 87-108
- **MERCURIO, A.M.** Laminin receptors: Achieving specificity through cooperation. *Trends Cell Biol.,* 1995, vol. 5, 419-423
- **MEYERSON, M. ; C. M. COUNTER ; E. N. EATON ; L. W. ELLISEN ; P. STEINER ; S. D. CADDLE ; L. ZIAUGRA ; R. L. BEIJERSBERGEN ; M. J. DAVIDOFF ; Q. LIU.** hEST2, the putative human telomerase catalytic subunit gene, is upregulated in tumor cells and during immortalization. *Cell,* 1997, vol. 90, 785-795
- **MIWA, S. ; CZAPIEWSKI, R. ; WAN, T ; BELL, A. ; HILL, K.N ; VON ZGLINICKI, T. ; SARETZKI, G.** Decreased mTOR signalling reduces mitochondrial ROS in brain via accumulation of the telomerase protein TERT within mitochondria. *Aging.,* 2016, vol. 8 (10), 2551-2567
- **MOODLEY, K. ; WEISS, S.F.T.** Downregulation of the non-integrin laminin receptor reduces cellular viability by inducing apoptosis in lung and cervical cancer cells. *PLoS One.,* 2013, vol. 8 (3), e57409
- **NAIDOO, K. ; MALINDISA, S.T. ; OTGAAR. T.C ; BERNERT, M. ; DA COSTA DIAS, B. ; FERREIRA, E. ; REUSCH, U. ; KNACKMUSS, S. ; LITTLE, M. ; WEISS, S.F.T.** Knock-Down of the 37kDa/67kDa Laminin Receptor LRP/LR Impedes Telomerase Activity. *PLoS One,* 2015, vol. 10 (11), e0141618
- **O'SULLIVAN, R.J. ; KARLSEDER, J.** Telomeres: protecting chromosomes against genome instability. *Nat Rev Mol Cell Biol.,* 2010, vol. 11 (3), 171-181
- **OTGAAR, T.C. ; FERREIRA, E. ; MALINDISA, S.T. ; BERNERT, M. ; LETSOLO, B.T. ; S.F.T. WEISS.** 37 kDa LRP::FLAG enhances telomerase activity and reduces senescent markers in vitro. *Oncotarget,* 2017
- **PALMER, A.K ; TCHKONIA, T ; LEBRASSEUR, N.K. ; CHINI, E.N. ; XU, M. ; KIRKLAND, J.L.** Cellular senescence in type 2 diabetes: A therapeutic opportunity. *Diabetes.,* 2015, vol. 65 (64), 2289-2298
- **RYU, J.M. ; LEE, H.J. ; JUNG, Y.H. ; LEE, K.H. ; KIM, D.I. ; KIM, J.Y ; KO, S.H. ; CHOI, G.E. ; CHAI, I.I ; SONG, E.J.** Regulation of Stem Cell Fate by ROS-mediated Alteration of Metabolism. *Int J Stem Cells.,* 2015, vol. 8 (1), 24-35
- **SALPEA, K.D. ; HUMPHRIES.** Telomere length in atherosclerosis and diabetes. *Atherosclerosis.,* 2010, vol. 209, 35-39
- **SANTOS, J.H. ; MEYER, J.N. ; VAN HOUTEN, B.** Mitochondrial localization of telomerase as a determinant for hydrogen peroxide- induced mitochondrial DNA damage and apoptosis. *Human Molecular Genetics.,* 2006, vol. 15 (11), 1757-1768
- **SAVAGE, D.B. ; PETERSEN, K.F ; SHULMAN, G.I.** Disordered lipid metabolism and the pathogenesis of insulin resistance. *Physiol Rev,* 2007, vol. 87, 507-520

- **SHAY, J.W ; ZOU, Y. ; HIYAMA, E. ; WRIGHT, W.E.** Telomerase and cancer. *Human Molecular Genetics.,* 2001, vol. 10 (7), 677-685
- **TOMAS-LOBA, A. ; FLORES, I. ; FERNA, P.J. ; CAYUELA, L. ; MARAVER, A. ; SERRANO, M ; TEJERA, A. ; BORRA, C. ; MATHEU, A. ; KLATT, P. et al.** Telomerase Reverse Transcriptase Delays Aging in Cancer-Resistant Mice. *Cell,* 2008, vol. 135, 609-622
- **VAN STEENSEL, B. ; SMOGORZEWSKA, A. ; DE LANGE, T.** TRF2 protects human telomeres from end-to-end fusions. *Cell,* 1998, vol. 92, 401-413
- **VAN DEURSEN, M.** The role of senescent cells in ageing. *Nature,* 2014, vol. 509 (7501), 439-446
- **VON ZGLINICKI, T. ; SARETZKI, G. ; DOCKE, W. ; LOTZE, C.** Mild hyperoxia shortens telomeres and inhibits proliferation of fibroblasts: a model for senescence?. *Exp Cell Res.,* 1995, vol. 220, 186-193
- **VON ZGLINICKI, T.** Oxidative stress shortens telomeres. *Trends Biochem. Sci.,* 2002, vol. 27 (7), 339-344
- **WEINRICH, S.L. ; PRUZAN, R ; MA, L. ; OUELLETTE, M. ; TESMER, V.M. ; HOLT, S.E. ; BODNAR, A.G ; LICHTSTEINER, S. ; KIM, N.W. ; TRAGER, J.B.** Reconstitution of human telomerase with the template RNA component hTR and the catalytic protein subunit hTRT. *Nat Genet.,* 1997, vol. 17, 498-502
- **WEISS, S.F.T.** Bad Boy with a Twist: Targeting the 37kda/67 KDa Laminin Receptor for Treatment of Cancer and Neurodegenerative Diseases and for Changing Telomere Dynamics. *Cell Cellular Lif Sci J.,* 2017, vol. 2 (2), 000114
- **WILCOX, G.** Insulin and Insulin resistance. *Clin Biochem Rev.,* 2005, vol. 26 (2), 19-39
- **XU, Y.** Chemistry in human telomere biology: structure, function and targeting of telomere DNA/RNA. *Chem. Soc. Rev.,* 2011, vol. 40, 2719-2740
- **ZHAO, J. ; ZHU, Y. ; LIN J. ; MATSUGUCHI, T ; BLACKBUM, E. ; ZHANG, Y. ; COLE, S.A. ; BEST, L.G. ; LEE, E.T. ; HOWARD,B.V.** Short leukocyte telomere length predicts risk of diabetes in American Indians: the Strong Heart Family Study. *Diabetes.,* 2013, vol. 63, 354-362